# EUROPEAN PATENT SPECIFICATION

(11) **EP 1 628 680 B1**
(45) Date of publication and mention of the grant of the patent: **24.10.2012**
(21) Application number: 04776023.6
(22) Date of filing: 17.05.2004
(51) Int. Cl.: A61K 39/21, C07H 21/04, C12P 21/06, C12N 15/63, C12N 5/00, C12N 15/00, C07K 14/16

(54) **HIV POLYNUCLEOTIDES AND POLYPEPTIDES DERIVED FROM BOTSWANA MJ4**
HIV POLYNUCLEOTIDE UND POLYPEPTIDE VON BOTSWANA MJ4
POLYNUCLEOTIDES POUR VIH ET POLYPEPTIDES DERIVES DE BOTSWANA MJ4

(30) Priority: 15.05.2003 US 471278 P
(43) Date of publication of application: 01.03.2006
(73) Proprietor: Novartis Vaccines and Diagnostics, Inc., Emeryville, CA 94608 (US)
(72) Inventor: LIAN, Ying, Emeryville, CA 94608 (US); ZUR MEGEDE, Jan, Emeryville, CA 94608 (US); SRIVASTAVA, Indresh, Everyville, CA 94608 (US); BARNETT, Susan W., Emeryville, CA 94608 (US)
(74) Representative: Marshall, Cameron John
(86) International application number: PCT/US2004/015431
(87) International publication number: WO 2005/007808

(56) References cited:
- WO-A-00/39302
- WO-A-01/60838
- WO-A-02/04493
- WO-A-02/26209
- US-A- 5 169 763
- US-A- 5 792 459
- US-A- 6 114 148
- US-B1- 6 197 755
- NDUNG'U T ET AL: "CONSTRUCTION AND ANALYSIS OF AN INFECTIOUS HUMAN IMMUNODEFICIENCY VIRUS TYPE 1 SUBTYPE C MOLECULAR CLONE" JOURNAL OF VIROLOGY, THE AMERICAN SOCIETY FOR MICROBIOLOGY, US, vol. 75, no. 11, 1 June 2001 (2001-06-01), pages 4964-4972, XP008061227 ISSN: 0022-538X
- NDUNG'U T. ET AL: 'Construction and analysis of an infectious human immunodeficiency virus type 1 subtype C molecular clon' J. VIROL. vol. 75, no. 11, June 2001, pages 4965 - 4972, XP008061226
- ANDRE S. ET AL: 'Increased immune response elicited by DNA vacination with a synthetic gp 120 sequence with optimized codon usage' J. VIROL. vol. 72, no. 2, February 1998, pages 1497 - 1503, XP002278034

## Description

### TECHNICAL FIELD

Polynucleotides encoding immunogenic Type C HIV Env polypeptides are described, as are uses of these polynucleotides and polypeptide products in immunogenic compositions.

### BACKGROUND

Acquired immune deficiency syndrome (AIDS) is recognized as one of the greatest health threats facing modem medicine. There is, as yet, no cure for this disease. In 1983-1984, three groups independently identified the suspected etiological agent of AIDS. See, e.g., Barre-Sinoussi et al. (1983) Science 220:868-871; Montagnier et al., in Human T-Cell Leukemia Viruses (Gallo, Essex & Gross, eds., 1984); Vilmer et al. (1984) The Lancet 1:753; Popovic et al. (1984) Science 224:497-500; Levy et al. (1984) Science 225:840-842. These isolates were variously called lymphadenopathy-associated virus (LAV), human T-cell lymphotropic virus type III (HTLV-III), or AIDS-associated retrovirus (ARV). All of these isolates are strains of the same virus, and were later collectively named Human Immunodeficiency Virus (HIV). With the isolation of a related AIDS-causing virus, the strains originally called HIV are now termed HIV-1 and the related virus is called HIV-2 See, e.g., Guyader et al. (1987) Nature 326:662-669; Brun-Vezinet et al. (1986) Science 233:343-346; Clavel et al. (1986) Nature 324:691-695.

A great deal of information has been gathered about the HIV virus, however, to date an effective vaccine has not been identified. HIV Env polypeptides in immunogenic compositions have also been described. (See, U.S. Patent No. 5,846,546 to Hurwitz et al., issued December 8, 1998, describing immunogenic compositions comprising a mixture of at least four different recombinant virus that each express a different HIV Env variant; and U.S. Patent No. 5,840,313 to Vahlne et al., issued November 24, 1998, describing peptides which correspond to epitopes of the HIV-1 gp120 protein). In addition, U.S. Patent No. 5,876,731 to Sia et al, issued March 2, 1999 describes candidate vaccines against HIV comprising an amino acid sequence of a T-cell epitope of Gag linked directly to an amino acid sequence of a B-cell epitope of the V3 loop protein of an HIV-1 isolate containing the sequence GPGR.

Further, although certain studies have demonstrated the presence of neutralizing antibodies during the acute phase of infection (Ruppach et al. (2000) J Virol 74(12):5403-11), it is accepted that the emergence of neutralizing antibody responses generally follows that of CTL responses (Lewis et al. (1998) J. Virol. 72:8943-8951; Moore et al. (1994) J. Virol. 68:5142-5155; Moore et al. (1993) J. Virol. 67:863-875). Neutralizing antibodies represent only a small fraction of the total anti-envelope antibodies circulating in the blood of humans infected with HIV or macaques infected with SIV or SHIV at any given time during infection (Burton et al. (1997) Proc. Natl Acad. Sci. USA 94:10018-10031).

However, the important contribution of neutralizing antibodies in preventing the establishment of HIV, SIV and SHIV infection or delaying the onset of disease is highlighted by several studies. First, the emergence of neutralization-resistant viruses coincides or precedes the development of disease in infected animals (Bums (1993) J Virol. 67:4104-13; Cheng-Mayer et al. (1999) J. Virol. 73:5294-5300; Narayan et al. (1999) Virology 256:54-63). Second, the pre-infusion of high concentrations of potent neutralizing monoclonal antibodies (mAbs) in the blood circulation of macaques, chimpanzees and SCID mice prior to their challenge with HIV, SIV or SHIV viruses, offers protection or delays the onset of disease (Conley et al. (1996) J. Virol. 70:6751-6758; Emini et al. (1992) Nature (London) 355:728-730; Gauduin et al. (1997) Nat Med. 3:1389-93; Mascola et al. (1999) J Virol. 73:4009-18; Mascola et al. (2000) Nature Med. 6(2):207-210; Baba et al. (2000) Nature Med. 6(2):200-206). Similarly, infusion of neutralizing antibodies collected from the serum of HIV-1-infected chimpanzees to naïve pig-tailed macaques protects the latter animals from subsequent viral challenge by SHIV viruses (Shibata et al (1999) Nature Medicine 5:204-210). Thus, there remains a need for immunogenic HIV polypeptides, particularly Type C isolates.

Recently, polynucleotides encoding antigenic HIV polypeptides and uses of these polynucleotides and polypeptides have been described. See, e.g., U.S. Patent No. 6,689,879 and 6,602,705; International Publications WO 00/39303, WO 00/39302, WO 00/39304, WO 02/04493, WO 03/004620, WO 03/004657, and WO 01/60838. Ndung'u et al. (2001) J. Virol. 75:4964-4972 describes the construction of a chimeric molecular clone of HIV-1 subtype C.

### SUMMARY

Described herein are novel Type C HIV sequences, polypeptides encoded by these novel sequences, and synthetic expression cassettes generated therefrom.

The present invention is defined in the claims.

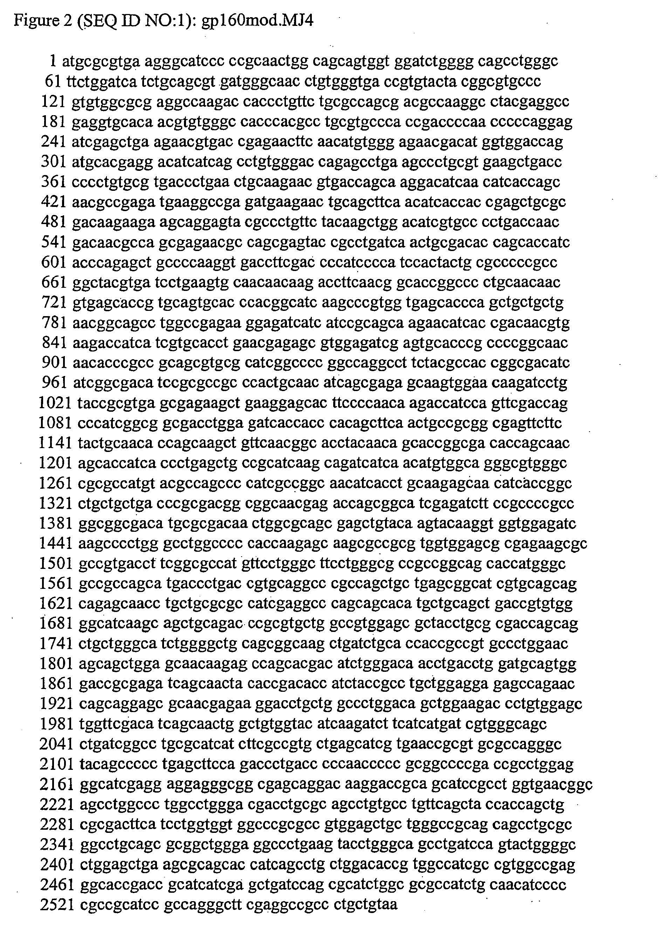

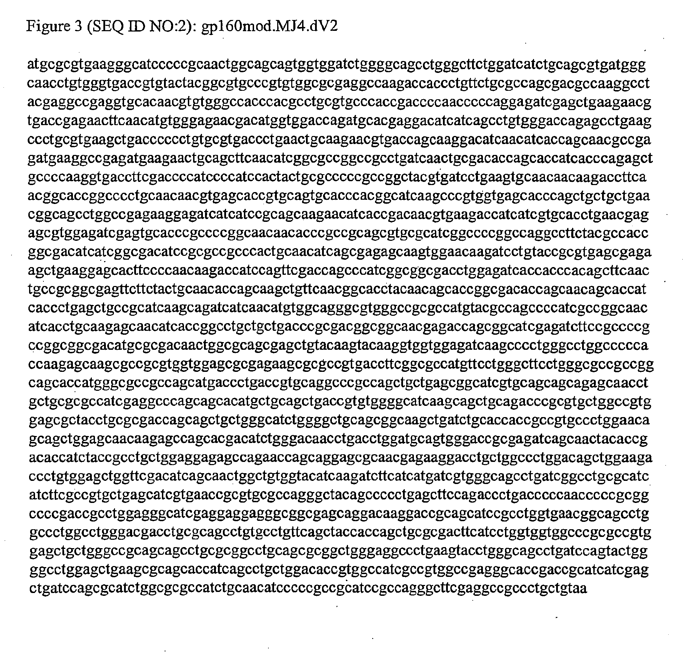

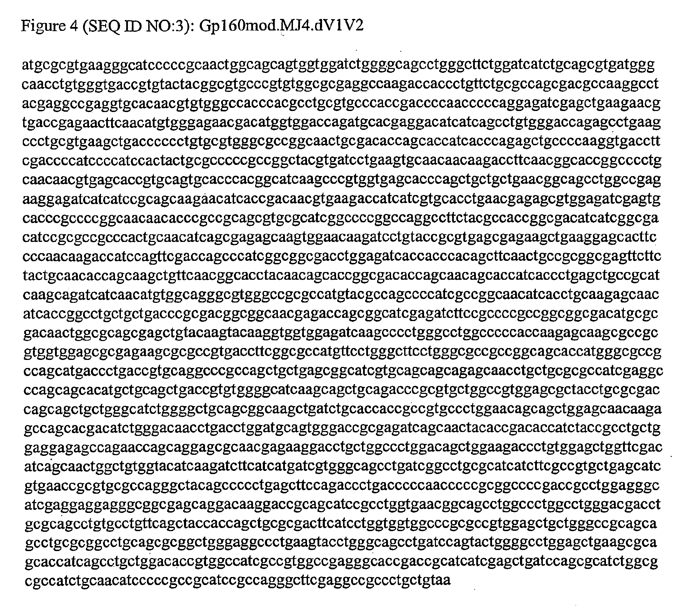

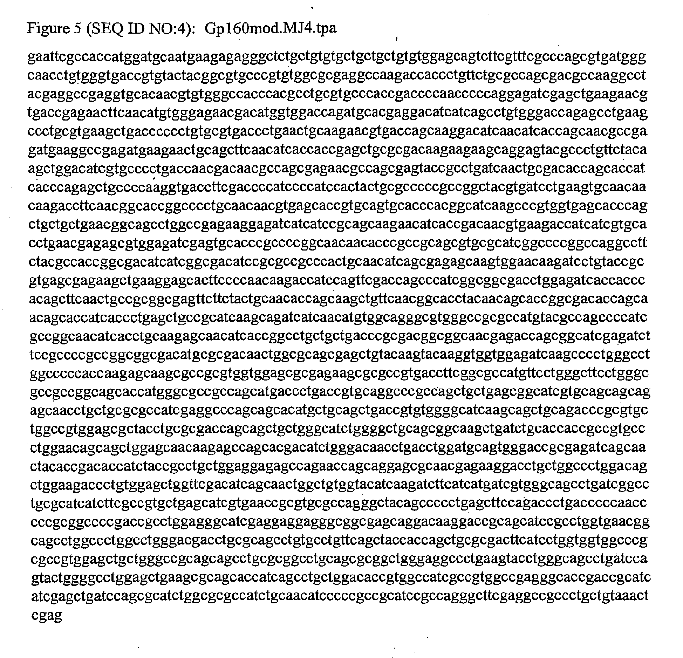

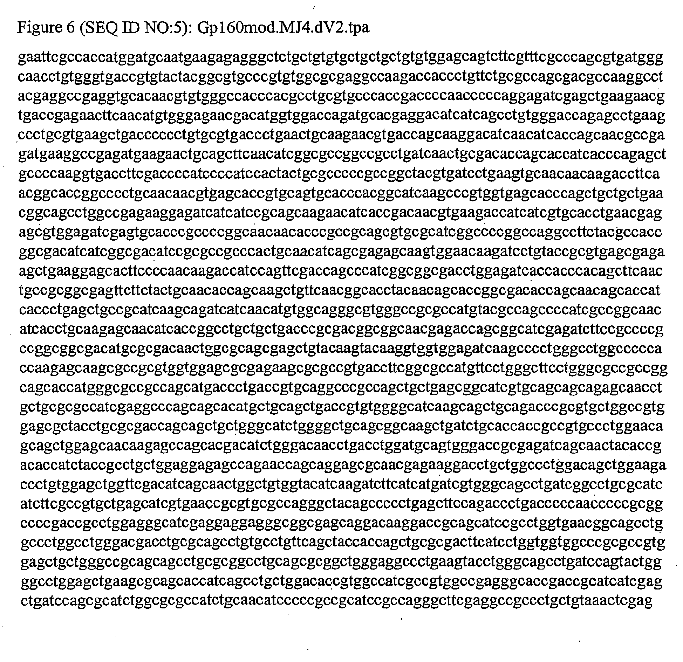

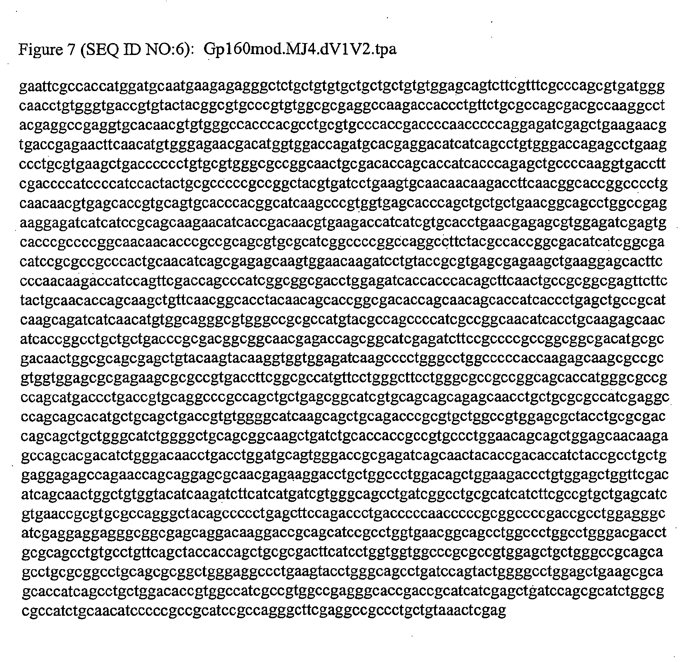

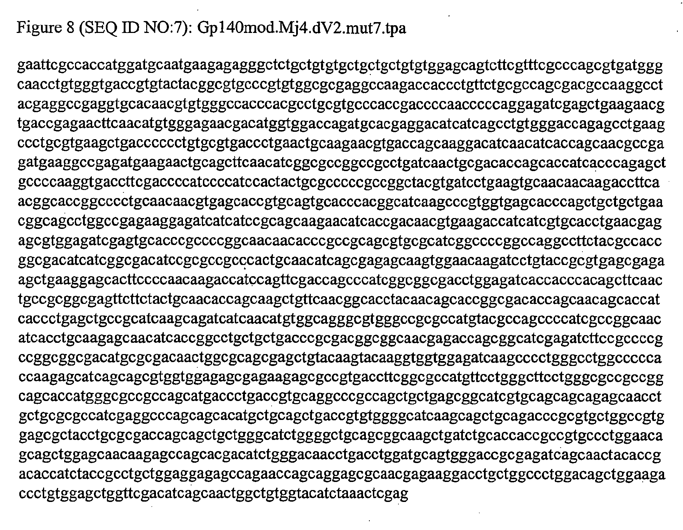

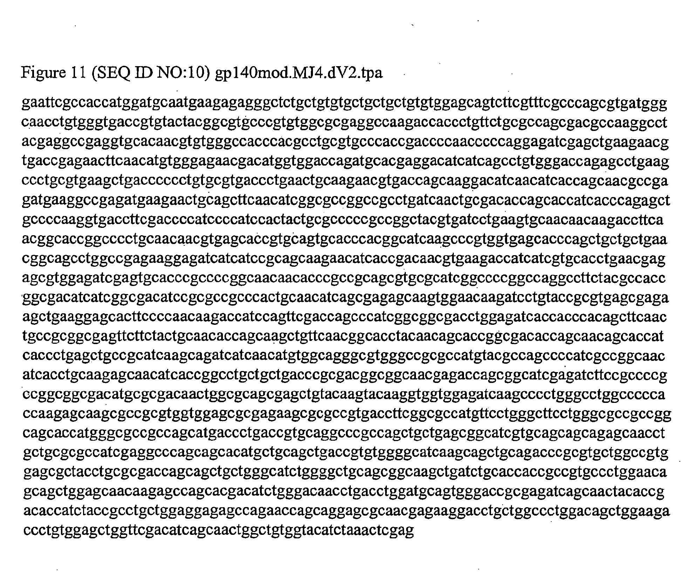

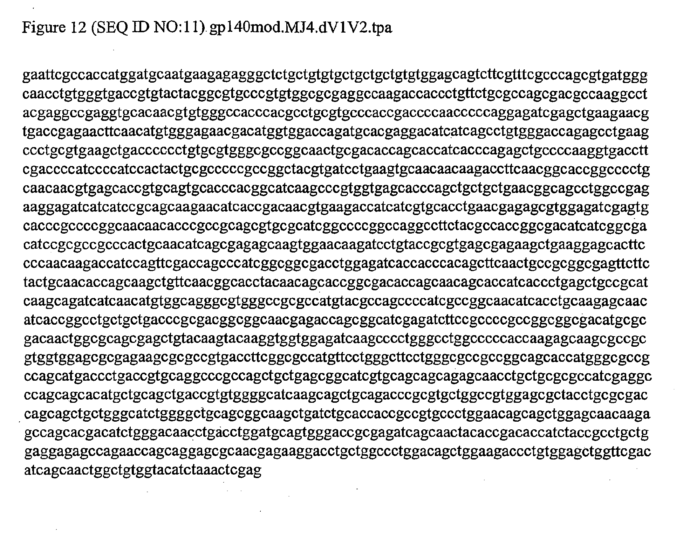

Disclosed herein is a polynucleotide sequence encoding one or more Env-containing polypeptides (*e.g*., immunogenic Env polypeptide), wherein the polynucleotide sequence comprises a sequence having at least about 85%, preferably about 90%, more preferably about 95%, and more preferably about 98% sequence (and any integers between these values) identity to the sequences taught in the present specification or fragments (*e.g*., gp120- or gp140-encoding fragments of gp160-encoding sequences described herein) of these sequences that encode immunogenic peptides. The polynucleotide sequences encoding Env-containing polypeptides include, but are not limited to, any of those depicted in figures 2-8, 10-12 and/or fragments of this sequences that encode a polypeptide that elicits an HIV *Env*-specific immune response.

Any of the polynucleotides may be inserted into a vector, for example, an expression cassette. The expression cassettes typically include an HIV-polypeptide encoding sequence inserted into an expression vector backbone.

The polynucleotides encoding the HIV polypeptides of the present invention may also include sequences encoding additional polypeptides. Such additional polynucleotides encoding polypeptides may include, for example, coding sequences for other viral proteins (*e.g*., hepatitis B or C or other HIV proteins, such as, polynucleotide sequences encoding an HIV Gag polypeptide, polynucleotide sequences encoding an HIV Pol polypeptide and/or polynucleotides encoding one or more of vif, vpr, tat, rev, vpu and nef); cytokines or other transgenes. In addition, sequences encoding Env polypeptides from other HIV subtypes and/or variants (*e.g*., A, B and/or other variants of C) can also be included.

Thus, polynucleotide sequences described herein typically encode a polypeptide including an HIV Env-containing polypeptide, wherein the polynucleotide sequence encoding the Env polypeptide comprises a sequence having at least about 85%, preferably about 90%, more preferably about 95%, and most preferably about 98% sequence identity to the sequences taught in the present specification. The polynucleotide sequences encoding Env-containing polypeptides include, but are not limited to, the following polynucleotides: SEQ ID NO:1-7. In certain aspects, the Env-encoding sequences will contain further modifications, for instance mutation of the cleavage site to prevent the cleavage of a gp160 polypeptide into a gp120 polypeptide and a gp41 polypeptide and/or deletion of variable regions (*e.g*., V1 and/or V2). Any of the sequences described herein preferably encode a polypeptide that elicits an HIV *Env*-specific immune response.

Native and synthetic polynucleotide sequences encoding the HIV polypeptides of the present disclosure typically have at least about 85%, preferably about 90%, more preferably about 95%, and most preferably about 98% sequence identity to the sequences taught herein. Further, in certain aspects, the polynucleotide sequences encoding the HIV polypeptides of the disclosure will exhibit 100% sequence identity to the sequences taught herein.

The polynucleotides of the present invention can be produced by recombinant techniques, synthetic techniques, or combinations thereof.

The present invention further includes recombinant expression systems for use in selected host cells, wherein the recombinant expression systems employ one or more of the polynucleotides and/or vectors (*e.g*., expression cassettes) of the present invention. In such systems, the polynucleotide sequences are preferably operably linked to control elements compatible with expression in the selected host cell. Numerous expression control elements are known to those in the art, including, but not limited to, the following: transcription promoters, transcription enhancer elements, transcription termination signals, polyadenylation sequences, sequences for optimization of initiation of translation, and translation termination sequences. Exemplary transcription promoters include, but are not limited to those derived from CMV, CMV+intron A, SV40, RSV, HIV-Ltr, MMLV-1tr, and metallothionein.

In another aspect the invention includes cells comprising one or more of the polynucleotide sequences of the invention, for example cells comprising vectors (*e.g*., expression cassettes) comprising the polynucleotide sequences, where the polynucleotide sequences are operably linked to control elements compatible with expression in the selected cell. In one embodiment such cells are mammalian cells. Exemplary mammalian cells include, but are not limited to, BHK, VERO, HT1080, 293, RD, COS-7, and CHO cells. Other cells, cell types, tissue types, etc., that may be useful in the practice of the present invention include, but are not limited to, those obtained from the following: insects (e.g., Trichoplusia ni (Tn5) and Sf9), bacteria, yeast, plants, antigen presenting cells (e.g., macrophage, monocytes, dendritic cells, B-cells, T-cells, stem cells, and progenitor cells thereof), primary cells, immortalized cells, tumor-derived cells.

In a further aspect, the present invention includes compositions for generating an immunological response, where the composition typically comprises at least one of the synthetic sequences and/or vectors of the present invention and may, for example, contain combinations of sequences and/or vectors (*e.g*., one or more expression cassettes described herein with one or more expression cassettes encoding additional HIV polypeptides such as Gag, Pol, vpu, vpr, nef, vif, tat, and/or rev, particularly immunogenic). Such compositions may further contain an adjuvant or adjuvants. The compositions may also contain one or more HIV polypeptides. The Type C HIV polypeptides may correspond to the polypeptides encoded by the expression cassette(s) in the composition, or may be different from those encoded by the expression cassettes.

In another aspect the present disclosure includes methods of immunization of a subject by introducing into a subject any of the compositions described herein. Typically, the conditions are compatible with expression of the synthetic sequence(s) in the subject. In certain aspects, the sequences are introduced as plasmids (*e.g*., using electroporation). In other aspects, the polynucleotides (and/or vectors containing the polynucleotides) of the present invention can be introduced using a gene delivery vector. The gene delivery vector can, for example, be a non-viral vector or a viral vector. Exemplary viral vectors include, but are not limited to alphavirus derived vectors (*e.g*., Sindbis-derived), retroviral vectors, and lentiviral vectors. Compositions useful for generating an immunological response can also be delivered using a particulate carrier, for example poly(lactide-co-glycolides), known as PLG. Further, such compositions can be coated on, for example, gold or tungsten particles and the coated particles delivered to the subject using, for example, a gene gun. The compositions can also be formulated as liposomes. In one aspect of this method, the subject is a mammal and can, for example, be a human.

In a further aspect, the disclosure includes methods of generating an immune response in a subject. Any of the sequences and/or vectors described herein can be expressed in a suitable cell to provide for the expression of the Type C HIV polypeptides encoded by the polynucleotides of the present invention. The polypeptide(s) are then isolated (e.g., substantially purified) and administered to the subject in an amount sufficient to elicit an immune response. In certain aspects, the methods comprise administration of one or more of the expression cassettes or polynucleotides of the present invention, using any of the gene delivery techniques described herein. In other aspects, the methods comprise co-administration of one or more of the polynucleotides and/or vectors of the present invention and one or more polypeptides, wherein the polypeptides can be expressed from these polynucleotides or can be other subtype C HIV polypeptides. In other aspects, the methods comprise co-administration of multiple polynucleotides and/or vectors of the present invention. In still further aspects, the methods comprise co-administration of multiple polypeptides, for example polypeptides expressed from the polynucleotides of the present invention and/or other subtype C HIV polypeptides.

The disclosure further includes methods of generating an immune response in a subject, where cells of a subject are transfected with any of the above-described polynucleotides of the present invention, under conditions that permit the expression of a selected polynucleotide and production of a polypeptide of interest (*e.g*., encoded by any expression cassette of the present invention). By this method an immunological response to the polypeptide is elicited in the subject. Transfection of the cells may be performed ex vivo and the transfected cells are reintroduced into the subject. Alternately, or in addition, the cells may be transfected in vivo in the subject. The immune response may be humoral and/or cell-mediated (cellular). The immune response may also be adaptive and/or innate. In a further aspect, this method may also include administration of a Type C HIV polypeptides before, concurrently with, and/or after introduction of the polynucleotides and/or vectors into the subject.

### Brief Description of the Figures

Figure 1 shows exemplary mutations in cleavage sites. Sequence is shown relative to MJ4 wild type.
Figure 2 (SEQ ID NO:1) depicts an exemplary Env-encoding sequence designated gp160mod.MJ4, which is a synthetic sequence of Env gp160 derived from wild-type MJ4.
Figure 3 (SEQ ID NO:2) depicts an exemplary Env-encoding sequence designated gp160mod.MJ4.dV2, which is synthetic sequence of Env gp160 derived from wild-type MJ4. The Env protein encoded by this sequence has the V2 region deleted.
Figure 4 (SEQ END NO:3) depicts an exemplary Env-encoding sequence designated gp160mod.MJ4.dV1V2, which is synthetic sequence of Env gp160 derived from wild-type MJ4. The Env protein encoded by this sequence has both the V1 and V2 regions deleted.
Figure 5 (SEQ ID NO:4) depicts an exemplary Env-encoding sequence designated gp160mod.MJ4.tpa, which is synthetic sequence of Env gp160 derived from wild-type MJ4. The sequence includes a native tpa leader sequence.
Figure 6 (SEQ ID NO:5) depicts an exemplary Env-encoding sequence designated 160mod.MJ4.dV2.tpa, which is synthetic sequence of Env gp160 derived from wild-type MJ4. The Env protein encoded by this sequence has the V2 region deleted and includes a native tpa leader sequence.
Figure 7 (SEQ ID NO:6) depicts an exemplary Env-encoding sequence designated gp160mod.MJ4.dV1V2.tpa, which is synthetic sequence of Env gp160 derived from wild-type MJ4. The Env protein encoded by this sequence has both V1 and V2 regions deleted and includes a modified tpa leader sequence.
Figure 8 (SEQ ID NO:7) depicts an exemplary Env-encoding sequence designated gp140mod.MJ4.dV2.mut7.tpa, which is synthetic sequence of Env gp140 derived from wild-type MJ4. The Env protein encoded by this sequence has the V2 region deleted, a modification to the protease cleavage region and includes a modified tpa leader sequence.
Figure 9 (SEQ ID NO:8) depicts the wild-type MJ4 Env gp160-encoding sequence.
Figure 10 (SEQ ID NO:9) depicts an exemplary Env-encoding sequence designated gp140mod.MJ4.tpa, which is a synthetic sequence of Env gp140 derived from wild-type MJ4. The Env protein encoded by this sequence includes a tpa leader sequence.
Figure 11 (SEQ ID NO:10) depicts an exemplary Env-encoding sequence designated gp140mod.MJ4.tpa.dV2, which is a synthetic sequence of Env gp140 derived from wild-type MJ4. The Env protein encoded by this sequence has the V2 region deleted and includes a tpa leader sequence.
Figure 12 (SEQ ID NO:11) depicts an exemplary Env-encoding sequence designated gp 140mod.MJ4.tpa.dV1V2, which is a synthetic sequence of Env gp140 derived from wild-type MJ4. The Env protein encoded by this sequence has the V1 and V2 regions deleted and includes a tpa leader sequence.
Figure 13 depicts an alignment of amino acid sequences of a portion of HIV Env. The alignment shows V1 and V1V2 deletes as compared to wild-type MJ4 (top line). In constructs encoding variable region deletes the amino acid triplet GAG was inserted to help maintain conformation of the Env protein. The V2 delete (middle line) is encoded by a sequence in which nucleotides 466-571 of Figure 6 are deleted and the V1V2 delete (bottom line) is encoded by a sequence in which nucleotides 372-580 of Figure 6 are deleted.
Figure 14 depicts N-glycosylation sites in an MJ4 Env (gp60) amino acid sequence. The 28 sites are shown in bold and are underlined. Modifications of one or more of these sites are contemplated.
Figure 15, pages 1 through 3, depict an alignment of gp160 amino acid sequences from MJ4 and SF162 along with a consensus sequence. Arrows indicate the beginning and end of the regions of β2/V1V2/β3 or β20/β21.

### Detailed Description

The practice of the present invention will employ, unless otherwise indicated, conventional methods of chemistry, biochemistry, molecular biology, immunology and pharmacology, within the skill of the art. Such techniques are explained fully in the literature. See, e.g., Remington's Pharmaceutical Sciences, 18th Edition (Easton, Pennsylvania: Mack Publishing Company, 1990); Methods In Enzymology (S. Colowick and N. Kaplan, eds., Academic Press, Inc.); and Handbook of Experimental Immunology, Vols. I-IV (D.M. Weir and C.C. Blackwell, eds., 1986, Blackwell Scientific Publications); Sambrook, et al., Molecular Cloning: A Laboratory Manual (2nd Edition, 1989); Short Protocols in Molecular Biology, 4th ed. (Ausubel et al. eds., 1999, John Wiley & Sons); Molecular Biology Techniques: An Intensive Laboratory Course, (Ream et al., eds., 1998, Academic Press); PCR (Introduction to Biotechniques Series), 2nd ed. (Newton & Graham eds., 1997, Springer Verlag).

As used in this specification and the appended claims, the singular forms "a," "an" and "the" include plural references unless the content clearly dictates otherwise. Thus, for example, reference to "an antigen" includes a mixture of two or more such agents.

### 1. Definitions

In describing the present invention, the following terms will be employed, and are intended to be defined as indicated below.

"Synthetic" sequences, as used herein, refers to HIV polypeptide-encoding polynucleotides whose expression has been modified as described herein, for example, by codon substitution and inactivation of inhibitory sequences. "Wild-type" or "native" sequences, as used herein, refers to polypeptide encoding sequences that are essentially as they are found in nature, e.g., Env encoding sequences as found in Type C isolate MJ4. The various regions of the HIV genome are shown in Table A, with numbering relative to MJ4. Thus, the term "Env" refers to one or more of the following polypeptides: gp160, gp140 and/or gp120.

An "antigen" refers to a molecule containing one or more epitopes (either linear, conformational or both) that will stimulate a host's immune system to make a humoral and/or cellular antigen-specific response. The term is used interchangeably with the term "immunogen." Normally, a B-cell epitope will include at least about 5 amino acids but can be as small as 3-4 amino acids. A T-cell epitope, such as a CTL epitope, will include at least about 7-9 amino acids, and a helper T-cell epitope at least about 12-20 amino acids. Normally, an epitope will include between about 7 and 15 amino acids, such as, 9, 10, 12 or 15 amino acids. The term "antigen" denotes both subunit antigens, (i.e., antigens which are separate and discrete from a whole organism with which the antigen is associated in nature), as well as, killed, attenuated or inactivated bacteria, viruses, fungi, parasites or other microbes. Antibodies such as anti-idiotype antibodies, or fragments thereof, and synthetic peptide mimotopes, which can mimic an antigen or antigenic determinant, are also captured under the definition of antigen as used herein. Similarly, an oligonucleotide or polynucleotide that expresses an antigen or antigenic determinant in vivo, such as in gene therapy and DNA immunization applications, is also included in the definition of antigen herein.

For purposes of the present invention, immunogens can be derived from any of several known viruses, bacteria, parasites and fungi, as described more fully below, for example immunogens derived from an HIV. Furthermore, for purposes of the present invention, an "immunogen" refers to a protein that includes modifications, such as deletions, additions and substitutions (generally conservative in nature), to the native sequence, so long as the protein maintains the ability to elicit an immunological response, as defined herein. These modifications may be deliberate, as through site-directed mutagenesis, or may be accidental, such as through mutations of hosts that produce the antigens. By "immunogenic fragment" is meant a fragment of an HIV polypeptide that includes one or more epitopes and thus elicits one or more of the immunological responses described herein. Such fragments can be identified by, e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Pat. No. 4,708,871; Geysen et al. (1984) Proc. Natl. Acad. Sci. USA 81:3998-4002; Geysen et al. (1986) Molec. Immunol. 23:709-715.

An "immunological response" to an antigen or composition is the development in a subject of a humoral and/or a cellular immune response to an antigen present in the composition of interest. For purposes of the present invention, a "humoral immune response" refers to an immune response mediated by antibody molecules, while a "cellular immune response" is one mediated by T-lymphocytes and/or other white blood cells. One important aspect of cellular immunity involves an antigen-specific response by cytolytic T-cells ("CTL"s). CTLs have specificity for peptide antigens that are presented in association with proteins encoded by the major histocompatibility complex (MHC) and expressed on the surfaces of cells. CTLs help induce and promote the destruction of intracellular microbes, or the lysis of cells infected with such microbes. Another aspect of cellular immunity involves an antigen-specific response by helper T-cells. Helper T-cells act to help stimulate the function, and focus the activity of, nonspecific effector cells against cells displaying peptide antigens in association with MHC molecules on their surface. A "cellular immune response" also refers to the production of cytokines, chemokines and other such molecules produced by activated T-cells and/or other white blood cells, including those derived from CD4+ and CD8+ T-cells.

A composition or vaccine that elicits a cellular immune response may serve to sensitize a vertebrate subject by the presentation of antigen in association with MHC molecules at the cell surface. The cell-mediated immune response is directed at, or near, cells presenting antigen at their surface. In addition, antigen-specific T-lymphocytes can be generated to allow for the future protection of an immunized host.

The ability of a particular immunogen to stimulate a cell-mediated immunological response may be determined by a number of assays, such as by lymphoproliferation (lymphocyte activation) assays, CTL cytotoxic cell assays, or by assaying for T-lymphocytes specific for the antigen in a sensitized subject. Such assays are well known in the art. See, e.g., Erickson et al., J. Immunol. (1993) 151:4189-4199; Doe et al., Eur. J. Immunol. (1994) 24:2369-2376. Recent methods of measuring cell-mediated immune response include measurement of intracellular cytokines or cytokine secretion by T-cell populations, or by measurement of epitope specific T-cells (e.g., by the tetramer technique)(reviewed by McMichael, A.J., and O'Callaghan, C.A., J. Exp. Med. 187(9)1367-1371, 1998; Mcheyzer-Williams, M.G., et al, Immunol. Rev. 150:5-21, 1996; Lalvani, A., et al, J. Exp. Med. 186:859-865, 1997).

Thus, an immunological response as used herein may be one that stimulates the production of antibodies (*e.g*., neutralizing antibodies that block bacterial toxins and pathogens such as viruses entering cells and replicating by binding to toxins and pathogens, typically protecting cells from infection and destruction). The antigen of interest may also elicit production of CTLs. Hence, an immunological response may include one or more of the following effects: the production of antibodies by B-cells; and/or the activation of suppressor T-cells and/or δγ T-cells directed specifically to an antigen or antigens present in the composition or vaccine of interest. These responses may serve to neutralize infectivity, and/or mediate antibody-complement, or antibody dependent cell cytotoxicity (ADCC) to provide protection to an immunized host. Such responses can be determined using standard immunoassays and neutralization assays, well known in the art. (See, *e.g*., Montefiori et al. (1988) J. Clin Microbiol. 26:231-235; Dreyer et al. (1999) AIDS Res Hum Retroviruses (1999) 15(17):1563-1571).

An "immunogenic composition" is a composition that comprises an antigenic molecule where administration of the composition to a subject results in the development in the subject of a humoral and/or a cellular immune response to the antigenic molecule of interest. The immunogenic composition can be introduced directly into a recipient subject, such as by injection, inhalation, oral, intranasal and mucosal (e.g., intra-rectally or intra-vaginally) administration.

By "subunit vaccine" is meant a vaccine composition that includes one or more selected antigens but not all antigens, derived from or homologous to, an antigen from a pathogen of interest such as from a virus, bacterium, parasite or fungus. Such a composition is substantially free of intact pathogen cells or pathogenic particles, or the lysate of such cells or particles. Thus, a "subunit vaccine" can be prepared from at least partially purified (preferably substantially purified) immunogenic polypeptides from the pathogen, or analogs thereof. The method of obtaining an antigen included in the subunit vaccine can thus include standard purification techniques, recombinant production, or synthetic production.

"Substantially purified" general refers to isolation of a substance (compound, polynucleotide, protein, polypeptide, polypeptide composition) such that the substance comprises the majority percent of the sample in which it resides. Typically in a sample a substantially purified component comprises 50%, preferably 80%-85%, more preferably 90-95% of the sample. Teclmiques for purifying polynucleotides and polypeptides of interest are well-known in the art and include, for example, ion-exchange chromatography, affinity chromatography and sedimentation according to density.

A "coding sequence" or a sequence that "encodes" a selected polypeptide is a nucleic acid molecule that is transcribed (in the case of DNA) and translated (in the case of mRNA) into a polypeptide in vivo when placed under the control of appropriate regulatory sequences (or "control elements"). The boundaries of the coding sequence are determined by a start codon at the 5' (amino) terminus and a translation stop codon at the 3' (carboxy) terminus. A coding sequence can include, but is not limited to, cDNA from viral, prokaryotic or eukaryotic mRNA, genomic DNA sequences from viral or prokaryotic DNA, and even synthetic DNA sequences. A transcription termination sequence such as a stop codon may be located 3' to the coding sequence.

Typical "control elements", include, but are not limited to, transcription promoters, transcription enhancer elements, transcription termination signals, polyadenylation sequences (located 3' to the translation stop codon), sequences for optimization of initiation of translation (located 5' to the coding sequence), and translation termination sequences.

A "polynucleotide coding sequence" or a sequence that "encodes" a selected polypeptide, is a nucleic acid molecule that is transcribed (in the case of DNA) and translated (in the case of mRNA) into a polypeptide in vivo when placed under the control of appropriate regulatory sequences (or "control elements"). The boundaries of the coding sequence are determined by a start codon at the 5' (amino) terminus and a translation stop codon at the 3' (carboxy) terminus. Exemplary coding sequences are the modified viral polypeptide-coding sequences of the present invention. A transcription termination sequence may be located 3' to the coding sequence. Typical "control elements", include, but are not limited to, transcription regulators, such as promoters, transcription enhancer elements, transcription termination signals, and polyadenylation sequences; and translation regulators, such as sequences for optimization of initiation of translation, e.g., Shine-Dalgarno (ribosome binding site) sequences, Kozak sequences (i.e., sequences for the optimization of translation, located, for example, 5' to the coding sequence), leader sequences (heterologous or native), translation initiation codon (e.g., ATG), and translation termination sequences. In certain embodiments, one or more translation regulation or initiation sequences (e.g., the leader sequence) are derived from wild-type translation initiation sequences, i.e., sequences that regulate translation of the coding region in their native state. Wild-type leader sequences that have been modified, using the methods described herein, also find use in the present invention. Native or modified leader sequences can be from any source, for example other strains, variants and/or subtypes of HIV or non-HIV sources (*e.g*., tpa leader sequence exemplified herein). Promoters can include inducible promoters (where expression of a polynucleotide sequence operably linked to the promoter is induced by an analyte, cofactor, regulatory protein, etc.), repressible promoters (where expression of a polynucleotide sequence operably linked to the promoter is induced by an analyte, cofactor, regulatory protein, etc.), and constitutive promoters.

A "nucleic acid" molecule can include, but is not limited to, prokaryotic sequences, eukaryotic mRNA, cDNA from eukaryotic mRNA, genomic DNA sequences from eukaryotic (e.g., mammalian) DNA, and even synthetic DNA sequences. The term also captures sequences that include any of the known base analogs of DNA and RNA.

"Operably linked" refers to an arrangement of elements wherein the components so described are configured so as to perform their usual function. Thus, a given promoter operably linked to a coding sequence is capable of effecting the expression of the coding sequence when the proper enzymes are present. The promoter need not be contiguous with the coding sequence, so long as it functions to direct the expression thereof. Thus, for example, intervening untranslated yet transcribed sequences can be present between the promoter sequence and the coding sequence and the promoter sequence can still be considered "operably linked" to the coding sequence.

"Recombinant" as used herein to describe a nucleic acid molecule means a polynucleotide of genomic, cDNA, semisynthetic, or synthetic origin which, by virtue of its origin or manipulation: (1) is not associated with all or a portion of the polynucleotide with which it is associated in nature; and/or (2) is linked to a polynucleotide other than that to which it is linked in nature. The term "recombinant" as used with respect to a protein or polypeptide means a polypeptide produced by expression of a recombinant polynucleotide. "Recombinant host cells," "host cells," "cells," "cell lines," "cell cultures," and other such terms denoting prokaryotic microorganisms or eukaryotic cell lines cultured as unicellular entities, are used interchangeably, and refer to cells which can be, or have been, used as recipients for recombinant vectors or other transfer DNA, and include the progeny of the original cell which has been transfected. It is understood that the progeny of a single parental cell may not necessarily be completely identical in morphology or in genomic or total DNA complement to the original parent, due to accidental or deliberate mutation. Progeny of the parental cell which are sufficiently similar to the parent to be characterized by the relevant property, such as the presence of a nucleotide sequence encoding a desired peptide, are included in the progeny intended by this definition, and are covered by the above terms.

Techniques for determining amino acid sequence "similarity" are well known in the art. In general, "similarity" means the exact amino acid to amino acid comparison of two or more polypeptides at the appropriate place, where amino acids are identical or possess similar chemical and/or physical properties such as charge or hydrophobicity. A so-termed "percent similarity" then can be determined between the compared polypeptide sequences. Techniques for determining nucleic acid and amino acid sequence identity also are well known in the art and include determining the nucleotide sequence of the mRNA for that gene (usually via a cDNA intermediate) and determining the amino acid sequence encoded thereby, and comparing this to a second amino acid sequence. In general, "identity" refers to an exact nucleotide to nucleotide or amino acid to amino acid correspondence of two polynucleotides or polypeptide sequences, respectively.

Two or more polynucleotide sequences can be compared by determining their "percent identity." Two or more amino acid sequences likewise can be compared by determining their "percent identity." The percent identity of two sequences, whether nucleic acid or peptide sequences, is generally described as the number of exact matches between two aligned sequences divided by the length of the shorter sequence and multiplied by 100. An approximate alignment for nucleic acid sequences is provided by the local homology algorithm of Smith and Waterman, Advances in Applied Mathematics 2:482-489 (1981). This algorithm can be extended to use with peptide sequences using the scoring matrix developed by Dayhoff, Atlas of Protein Sequences and Structure, M.O. Dayhoff ed., 5 suppl. 3:353-358, National Biomedical Research Foundation, Washington, D.C., USA, and normalized by Gribskov, ucl. Acids Res. 14(6):6745-6763 (1986). An implementation of this algorithm for nucleic acid and peptide sequences is provided by the Genetics Computer Group (Madison, WI) in their BestFit utility application. The default parameters for this method are described in the Wisconsin Sequence Analysis Package Program Manual, Version 8 (1995) (available from Genetics Computer Group, Madison, WI). Other equally suitable programs for calculating the percent identity or similarity between sequences are generally known in the art.

For example, percent identity of a particular nucleotide sequence to a reference sequence can be determined using the homology algorithm of Smith and Waterman with a default scoring table and a gap penalty of six nucleotide positions. Another method of establishing percent identity in the context of the present invention is to use the MPSRCH package of programs copyrighted by the University of Edinburgh, developed by John F. Collins and Shane S. Sturrok, and distributed by IntelliGenetics, Inc. (Mountain View, CA). From this suite of packages, the Smith-Waterman algorithm can be employed where default parameters are used for the scoring table (for example, gap open penalty of 12, gap extension penalty of one, and a gap of six). From the data generated, the "Match" value reflects "sequence identity." Other suitable programs for calculating the percent identity or similarity between sequences are generally known in the art, such as the alignment program BLAST, which can also be used with default parameters. For example, BLASTN and BLASTP can be used with the following default parameters: genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + Swiss protein + Spupdate + PIR. Details of these programs can be found at the following internet address: http://www.ncbi.nlm.gov/cgi-bin/BLAST.

One of skill in the art can readily determine the proper search parameters to use for a given sequence, exemplary preferred Smith Waterman based parameters are presented above. For example, the search parameters may vary based on the size of the sequence in question. Thus, for the polynucleotide sequences of the present invention the length of the polynucleotide sequence disclosed herein is searched against a selected database and compared to sequences of essentially the same length to determine percent identity. For example, a representative embodiment of the present invention would include an isolated polynucleotide having X contiguous nucleotides, wherein (i) the X contiguous nucleotides have at least about a selected level of percent identity relative to Y contiguous nucleotides of the sequences described herein, and (ii) for search purposes X equals Y, wherein Y is a selected reference polynucleotide of defined length.

The sequences of the present disclosure can include fragments of the sequences, for example, from about 15 nucleotides up to the number of nucleotides present in the full-length sequences described herein (e.g., see the Sequence Listing, Figures, and claims), including all integer values falling within the above-described range. For example, fragments of the polynucleotide sequences of the present disclosure may be 30-60 nucleotides, 60-120 nucleotides, 120-240 nucleotides, 240-480 nucleotides, 480-1000 nucleotides, and all integer values therebetween.

The synthetic polynucleotides of the present disclosure include related polynucleotide sequences having about 80% to 100%, greater than 80-85%, preferably greater than 90-92%, more preferably greater than 92-95%, more preferably greater than 95%, and most preferably greater than 98% up to 100% (including all integer values falling within these described ranges) sequence identity to the synthetic polynucleotide sequences disclosed herein (for example, to the claimed sequences or other sequences of the present disclosure) when the sequences of the present disclosure are used as the query sequence against, for example, a database of sequences.

Two nucleic acid fragments are considered to "selectively hybridize" as described herein. The degree of sequence identity between two nucleic acid molecules affects the efficiency and strength of hybridization events between such molecules. A partially identical nucleic acid sequence will at least partially inhibit a completely identical sequence from hybridizing to a target molecule. Inhibition of hybridization of the completely identical sequence can be assessed using hybridization assays that are well known in the art (e.g., Southern blot, Northern blot, solution hybridization, or the like, see Sambrook, et al., *supra* or Ausubel et al., *supra*). Such assays can be conducted using varying degrees of selectivity, for example, using conditions varying from low to high stringency. If conditions of low stringency are employed, the absence of non-specific binding can be assessed using a secondary probe that lacks even a partial degree of sequence identity (for example, a probe having less than about 30% sequence identity with the target molecule), such that, in the absence of non-specific binding events, the secondary probe will not hybridize to the target.

When utilizing a hybridization-based detection system, a nucleic acid probe is chosen that is complementary to a target nucleic acid sequence, and then by selection of appropriate conditions the probe and the target sequence "selectively hybridize," or bind, to each other to form a hybrid molecule. A nucleic acid molecule that is capable of hybridizing selectively to a target sequence under "moderately stringent" typically hybridizes under conditions that allow detection of a target nucleic acid sequence of at least about 10-14 nucleotides in length having at least approximately 70% sequence identity with the sequence of the selected nucleic acid probe. Stringent hybridization conditions typically allow detection of target nucleic acid sequences of at least about 10-14 nucleotides in length having a sequence identity of greater than about 90-95% with the sequence of the selected nucleic acid probe. Hybridization conditions useful for probe/target hybridization where the probe and target have a specific degree of sequence identity, can be determined as is known in the art (see, for example, Nucleic Acid Hybridization: A Practical Approach, editors B.D. Hames and S.J. Higgins, (1985) Oxford; Washington, DC; IRL Press).

With respect to stringency conditions for hybridization, it is well known in the art that numerous equivalent conditions can be employed to establish a particular stringency by varying, for example, the following factors: the length and nature of probe and target sequences, base composition of the various sequences, concentrations of salts and other hybridization solution components, the presence or absence of blocking agents in the hybridization solutions (e.g., formamide, dextran sulfate, and polyethylene glycol), hybridization reaction temperature and time parameters, as well as, varying wash conditions. The selection of a particular set of hybridization conditions is selected following standard methods in the art (see, for example, Sambrook, et al., supra or Ausubel et al., supra).

A first polynucleotide is "derived from" second polynucleotide if it has the same or substantially the same basepair sequence as a region of the second polynucleotide, its cDNA, complements thereof, or if it displays sequence identity as described above.

A first polypeptide is "derived from" a second polypeptide if it is (i) encoded by a first polynucleotide derived from a second polynucleotide, or (ii) displays sequence identity to the second polypeptides as described above.

Generally, a viral polypeptide is "derived from" a particular polypeptide of a virus (viral polypeptide) if it is (i) encoded by an open reading frame of a polynucleotide of that virus (viral polynucleotide), or (ii) displays sequence identity to polypeptides of that virus as described above.

"Encoded by" refers to a nucleic acid sequence which codes for a polypeptide sequence, wherein the polypeptide sequence or a portion thereof contains an amino acid sequence of at least 3 to 5 amino acids, more preferably at least 8 to 10 amino acids, and even more preferably at least 15 to 20 amino acids from a polypeptide encoded by the nucleic acid sequence. Also encompassed are polypeptide sequences that are immunologically identifiable with a polypeptide encoded by the sequence. Further, polyproteins can be constructed by fusing in-frame two or more polynucleotide sequences encoding polypeptide or peptide products. Further, polycistronic coding sequences may be produced by placing two or more polynucleotide sequences encoding polypeptide products adjacent each other, typically under the control of one promoter, wherein each polypeptide coding sequence may be modified to include sequences for internal ribosome binding sites.

"Purified polynucleotide" refers to a polynucleotide of interest or fragment thereof that is essentially free, e.g., contains less than about 50%, preferably less than about 70%, and more preferably less than about 90%, of the protein with which the polynucleotide is naturally associated. Techniques for purifying polynucleotides of interest are well known in the art and include, for example, disruption of the cell containing the polynucleotide with a chaotropic agent and separation of the polynucleotide(s) and proteins by ion-exchange chromatography, affinity chromatography and sedimentation according to density.

By "nucleic acid immunization" is meant the introduction of a nucleic acid molecule encoding one or more selected antigens into a host cell, for the in vivo expression of an antigen, antigens, an epitope, or epitopes. The nucleic acid molecule can be introduced directly into a recipient subject, such as by injection, inhalation, oral, intranasal and mucosal administration, or the like, or can be introduced ex vivo, into cells which have been removed from the host. In the latter case, the transformed cells are reintroduced into the subject where an immune response can be mounted against the antigen encoded by the nucleic acid molecule.

"Gene transfer" or "gene delivery" refers to methods or systems for reliably inserting DNA of interest into a host cell. Such methods can result in transient expression of non-integrated transferred DNA, extrachromosomal replication and expression of transferred replicons (e.g., episomes), or integration of transferred genetic material into the genomic DNA of host cells. Gene delivery expression vectors include, but are not limited to, vectors derived from alphaviruses, pox viruses and vaccinia viruses. When used for immunization, such gene delivery expression vectors may be referred to as vaccines or vaccine vectors.

"T lymphocytes" or "T cells" are non-antibody producing lymphocytes that constitute a part of the cell-mediated arm of the immune system. T cells arise from immature lymphocytes that migrate from the bone marrow to the thymus, where they undergo a maturation process under the direction of thymic hormones. Here, the mature lymphocytes rapidly divide increasing to very large numbers. The maturing T cells become immunocompetent based on their ability to recognize and bind a specific antigen. Activation of immunocompetent T cells is triggered when an antigen binds to the lymphocyte's surface receptors.

The term "transfection" is used to refer to the uptake of foreign DNA by a cell. A cell has been "transfected" when exogenous DNA has been introduced inside the cell membrane. A number of transfection techniques are generally known in the art. See, e.g., Graham et al. (1973) Virology, 52:456, Sambrook et al. (1989) Molecular Cloning, a laboratory manual, Cold Spring Harbor Laboratories, New York, Davis et al. (1986) Basic Methods in Molecular Biology, Elsevier, and Chu et al. (1981) Gene 13:197. Such techniques can be used to introduce one or more exogenous DNA moieties into suitable host cells. The term refers to both stable and transient uptake of the genetic material, and includes uptake of peptide- or antibody-linked DNAs.

A "vector" is capable of transferring gene sequences to target cells (e.g., viral vectors, non-viral vectors, particulate carriers, and liposomes). Typically, "vector construct," "expression vector," and "gene transfer vector," mean any nucleic acid construct capable of directing the expression of a gene of interest and which can transfer gene sequences to target cells. Thus, the term includes cloning and expression vehicles, as well as viral vectors.

Transfer of a "suicide gene" (e.g., a drug-susceptibility gene) to a target cell renders the cell sensitive to compounds or compositions that are relatively nontoxic to normal cells. Moolten, F.L. (1994) Cancer Gene Ther. 1:279-287. Examples of suicide genes are thymidine kinase of herpes simplex virus (HSV-tk), cytochrome P450 (Manome et al. (1996) Gene Therapy 3:513-520), human deoxycytidine kinase (Manome et al. (1996) Nature Medicine 2(5):567-573) and the bacterial enzyme cytosine deaminase (Dong et al. (1996) Human Gene Therapy 7:713-720). Cells that express these genes are rendered sensitive to the effects of the relatively nontoxic prodrugs ganciclovir (HSV-tk), cyclophosphamide (cytochrome P450 2B1), cytosine arabinoside (human deoxycytidine kinase) or 5-fluorocytosine (bacterial cytosine deaminase). Culver et al. (1992) Science 256:1550-1552, Huber et al. (1994) Proc. Natl. Acad. Sci. USA 91:8302-8306.

A "selectable marker" or "reporter marker" refers to a nucleotide sequence included in a gene transfer vector that has no therapeutic activity, but rather is included to allow for simpler preparation, manufacturing, characterization or testing of the gene transfer vector.

A "specific binding agent" refers to a member of a specific binding pair of molecules wherein one of the molecules specifically binds to the second molecule through chemical and/or physical means. One example of a specific binding agent is an antibody directed against a selected antigen.

By "subject" is meant any member of the subphylum chordata, including, without limitation, humans and other primates, including non-human primates such as chimpanzees and other apes and monkey species; farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. The term does not denote a particular age. Thus, both adult and newborn individuals are intended to be covered. The system described above is intended for use in any of the above vertebrate species, since the immune systems of all of these vertebrates operate similarly.

By "pharmaceutically acceptable" or "pharmacologically acceptable" is meant a material which is not biologically or otherwise undesirable, i.e., the material may be administered to an individual in a formulation or composition without causing any undesirable biological effects or interacting in a deleterious manner with any of the components of the composition in which it is contained.

By "physiological pH" or a "pH in the physiological range" is meant a pH in the range of approximately 7.2 to 8.0 inclusive, more typically in the range of approximately 7.2 to 7.6 inclusive.

As used herein, "treatment" refers to any of (I) the prevention of infection or reinfection, as in a traditional vaccine, (ii) the reduction or elimination of symptoms, and (iii) the substantial or complete elimination of the pathogen in question. Treatment may be effected prophylactically (prior to infection) or therapeutically (following infection).

By "co-administration" is meant administration of more than one composition or molecule. Thus, co-administration includes concurrent administration or sequentially administration (in any order), via the same or different routes of administration. Non-limiting examples of co-administration regimes include, co-administration of nucleic acid and polypeptide; co-administration of different nucleic acids (e.g., different expression cassettes as described herein and/or different gene delivery vectors); and co-administration of different polypeptides (e.g., different HIV polypeptides and/or different adjuvants). The term also encompasses multiple administrations of one of the co-administered molecules or compositions (e.g., multiple administrations of one or more of the polynucleotides and/or expression cassettes described herein followed by one or more administrations of a polypeptide-containing composition). In cases where the molecules or compositions are delivered sequentially, the time between each administration can be readily determined by one of skill in the art in view of the teachings herein.

"Lentiviral vector", and "recombinant lentiviral vector" refer to a nucleic acid construct that carries, and within certain embodiments, is capable of directing the expression of a nucleic acid molecule of interest. The lentiviral vector include at least one transcriptional promoter/enhancer or locus defining element(s), or other elements which control gene expression by other means such as alternate splicing, nuclear RNA export, post-translational modification of messenger, or post-transcriptional modification of protein. Such vector constructs must also include a packaging signal, long terminal repeats (LTRS) or portion thereof, and positive and negative strand primer binding sites appropriate to the retrovirus used (if these are not already present in the retroviral vector). Optionally, the recombinant lentiviral vector may also include a signal that directs polyadenylation, selectable markers such as Neo, TK, hygromycin, phleomycin, histidinol, or DHFR, as well as one or more restriction sites and a translation termination sequence. By way of example, such vectors typically include a 5' LTR, a tRNA binding site, a packaging signal, an origin of second strand DNA synthesis, and a 3'LTR or a portion thereof

"Lentiviral vector particle" as utilized within the present invention refers to a lentivirus that carries at least one gene of interest. The retrovirus may also contain a selectable marker. The recombinant lentivirus is capable of reverse transcribing its genetic material (RNA) into DNA and incorporating this genetic material into a host cell's DNA upon infection. Lentiviral vector particles may have a lentiviral envelope, a non-lentiviral envelope (e.g., an ampho or VSV-G envelope), or a chimeric envelope. An "alphavirus vector" refers to a nucleic acid construct that carries, and within certain embodiments, is capable of directing the expression of a nucleic acid molecule of interest. Alphavirus vectors may be utilized in several formats, including DNA, RNA, and recombinant replicon particles. Such replicon vectors have been derived from alphaviruses that include, for example, Sindbis virus, Semliki Forest virus, and/or Venezuelan equine encephalitis virus. See, e.g., U.S. Patent Nos. 5,789,245; 5,814,482; and 6,376,235. The terms "alphavirus RNA replicon vector", "RNA replicon vector", "replicon vector" or "replicon" refer to an RNA molecule that is capable of directing its own amplification or self-replication *in vivo,* within a target cell. To direct its own amplification, the RNA molecule should encode the polymerase(s) necessary to catalyze RNA amplification (*e.g*., alphavirus nonstructural proteins nsP1, nsP2, nsP3, nsP4) and also contain *cis* RNA sequences required for replication which are recognized and utilized by the encoded polymerase(s). An alphavirus RNA vector replicon typically contains following ordered elements: 5' viral or cellular sequences required for nonstructural protein-mediated amplification (may also be referred to as 5' CSE, or 5' *cis* replication sequence, or 5' viral sequences required in *cis* for replication, or 5' sequence which is capable of initiating transcription of an alphavirus), sequences which, when expressed, code for biologically active alphavirus nonstructural proteins (*e.g*., nsP1, nsP2, nsP3, nsP4), and 3' viral or cellular sequences required for nonstructural protein-mediated amplification (may also be referred as 3' CSE, or 3' viral sequences required in *cis* for replication, or an alphavirus RNA polymerase recognition sequence). The alphavirus RNA vector replicon also should contain a means to express one or more heterologous sequence(s), such as for example, an IRES or a viral (*e.g*., alphaviral) subgeriomic promoter (*e.g*., junction region promoter) which may, in certain embodiments, be modified in order to increase or reduce viral transcription of the subgenomic fragment, or to decrease homology with defective helper or structural protein expression cassettes, and one or more heterologous sequence(s) to be expressed. When used as vectors, the replicons will also contain additional sequences, for example, one or more heterologous sequence(s) encoding one or more polypeptides (*e.g*., a protein-encoding gene or a 3' proximal gene) and/or a polyadenylate tract.

"Nucleic acid expression vector" or "Expression cassette" refers to an assembly that is capable of directing the expression of a sequence or gene of interest. The nucleic acid expression vector typically includes a promoter that is operably linked to the sequences or gene(s) of interest. Other control elements may be present as well. Expression cassettes described herein may be contained within a plasmid construct. In addition to the components of the expression cassette, the plasmid construct may also include a bacterial origin of replication, one or more selectable markers, a signal which allows the plasmid construct to exist as single-stranded DNA (e.g., a M13 origin of replication), a multiple cloning site, and a "mammalian" origin of replication (e.g., a SV40 or adenovirus origin of replication).

"Packaging cell" refers to a cell that contains those elements necessary for production of infectious recombinant viral that are lacking in a recombinant viral vector. Typically, such packaging cells contain one or more expression cassettes that are capable of expressing proteins that encode Gag, pol and/or Env proteins.

"Producer cell" or "vector producing cell" refers to a cell that contains all elements necessary for production of recombinant viral vector particles.

### 2. Modes of Carrying Out the Invention

Before describing the present invention in detail, it is to be understood that this invention is not limited to particular formulations or process parameters and such may, of course, vary. It is also to be understood that the terminology used herein is for the purpose of describing particular embodiments of the invention only, and is not intended to be limiting.

Although a number of methods and materials similar or equivalent to those described herein can be used in the practice of the present invention, the preferred materials and methods are described herein.

### 2.1. Overview

A fundamental criterion of an effective HIV vaccine is its ability to induce broad and potent neutralizing antibody responses against prevalent HIV strains. (See, *e.g*., Mascola et al. (1999) Virol. 73:4009-4018; Mascola et al. (2000) Nature Med. 6(2):207-210; Baba et al., supra). HIV-1 subtype C strains constitute more than 50% of the current HIV-infected populations and are mainly distributed in sub-Saharan African, India, and China. Numerous studies conducted in humans and animals have clearly demonstrated that the HIV envelope, when used as an immunogen is capable of eliciting the generation of high titer anti-envelope antibodies. However, in contrast to what occurs during infection, neutralizing antibodies are not readily developed during immunization with envelope-based immunogens, especially against those heterologous to the vaccine primary (PR) isolates (Hanson (1994) AIDS Res. Hum. Retroviruses 10:645-648; Mascola et al. (1999) J Virol. 73:4009-18 (45, 66). It appears therefore that a qualitative difference exists in the antibodies generated during infection and during vaccination. Without being bound by one theory, it appears there may be several potential reasons may account for this difference, including the inability of our current immunization protocols to elicit a maturation of the anti-envelope antibody responses in vaccines; the existence of structural differences between the envelope-immunogen and the functional envelope molecules present on the surface of infectious virions or infected cells; and the poor exposure of conserved neutralization epitopes on the vaccine immunogens.

Described herein are sequences encoding functional envelope genes derived from the infectious chimeric molecular clone, MJ4 (Ndhug'u et al. (2001) J. Virol. 75:4964-4972). MJ4's apparent use of the CCR5 co-receptor for virus entry along with its ability to grow to high titers in both primary peripheral blood mononuclear cells (PBMCs) and macrophage cultures make is a desirable starting point for the development of immunogenic compositions.

### 2.2. The HIV Genome

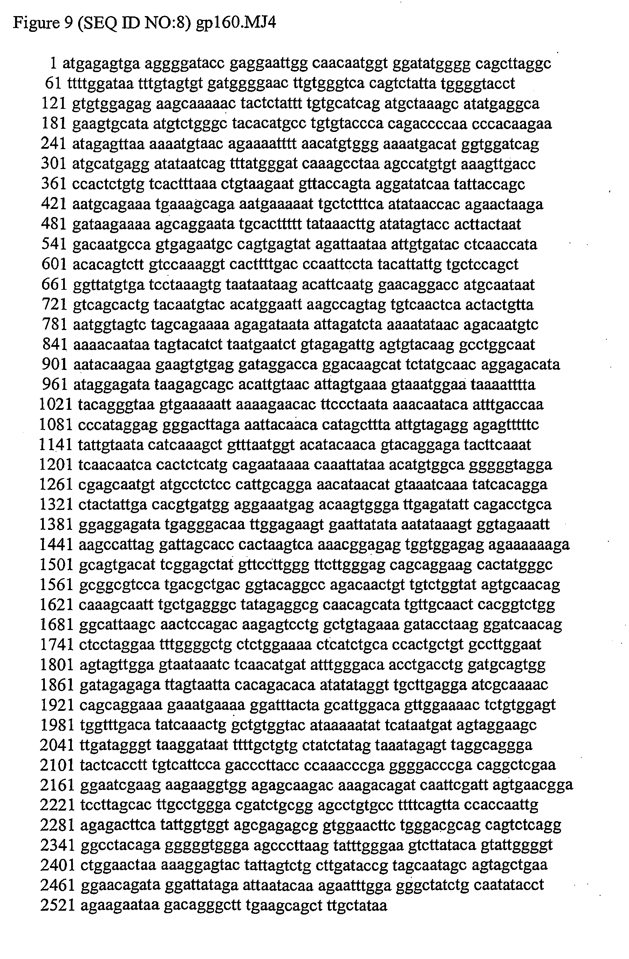

The HIV genome and various polypeptide-encoding regions are shown in Table A. The nucleotide positions are given relative to MJ4 (SEQ ID NO:8, Figure 9). However, it will be readily apparent to one of ordinary skill in the art in view of the teachings of the present disclosure how to determine corresponding regions in other HIV strains or variants (e.g., isolates HIVIIIb, HIVSF2, HIV-1SF162, HIV-1SF170, HIVLAV, HIVLAI, HIVMN, HIV-1CM235, HIV-1US4, MJ4, other HIV-1 strains from diverse subtypes(e.g., subtypes, A through G, and O), HIV-2 strains and diverse subtypes (e.g., HIV-2UC1 and HIV-2UC2), and simian immunodeficiency virus (SIV). (See, e.g., Virology, 3rd Edition (W.K. Joklik ed. 1988); Fundamental Virology, 2nd Edition (B.N. Fields and D.M. Knipe, eds. 1991); Virology, 3rd Edition (Fields, BN, DM Knipe, PM Howley, Editors, 1996, Lippincott-Raven, Philadelphia, PA; for a description of these and other related viruses), using for example, sequence comparison programs (e.g., BLAST and others described herein) or identification and alignment of structural features (e.g., a program such as the "ALB" program described herein that can identify the various regions).

**Table A: Regions of the HIV Genome relative to MJ4**

| **Region** | **Position in nucleotide sequence** |
|---|---|
| **5'LTR** | **1-632** |
| U3 | 1-456 |
| R | 457-552 |
| U5 | 553-635 |
| NFkB II | 354-363 |
| NFkB I | 367-376 |
| Sp1 III | 379-388 |
| Sp1 II | 390-399 |
| Sp1 I | 400-409 |
| TATA Box | 428-432 |
| TAR | 454-514 |
| Poly A signal | 528-533 |
| | |
| **PBS** | **636-654** |
| | |
| **p7 binding region, packaging signal** | **682-792** |
| | |

| **Gag:** | **793-2274** |
|---|---|
| p17 | 793-1180 |
| p24 | 1181-1872 |
| Cyclophilin A bdg. | 1396-1507 |
| MHR | 1633-1696 |
| p2 | 1873-1911 |
| p7 | 1912-2076 |
| Frameshift slip | 2076-2082 |
| p1 | 2077-2127 |
| p6Gag | 2128-2272 |
| Zn-motif I | 1954-1996 |
| Zn-motif II | 2017-2059 |
| | |

| **Pol:** | **2076-5078** |
|---|---|
| plp6Pol | 2079-2234 |
| Prot | 2235-2532 |
| P51RT | 2533-3852 |
| p66RT | 2533-4211 |
| p15RNaseH | 3853-4211 |
| p31Int | 4212-5077 |
| | |

| **Vif:** | **5023-5601** |
|---|---|
| Hydrophilic region | 5281-5304 |
| | |

| **Vpr:** | **5541-5831** |
|---|---|
| Oligomerization | 5541-5667 |
| Amphipathic α- helix | 5585-5641 |
| | |

| **Tat:** | **5812-6026 and 8349-8439** |
|---|---|
| Tat-1 exon | 5812-6026 |
| Tat-2 exon | 8349-8439 |
| N-terminal domain | 5812-5874 |
| Trans-activation domain | 5875-5923 |
| Transduction domain | 5950-5982 |
| | |

| **Rev:** | **5951-6026 and 8349-8596** |
|---|---|
| Rev-1 exon | 5962-6037 |
| Rev-2 exon | 8416-8663 |
| High-affinity bdg. site | 8372-8420 |
| Leu-rich effector domain | 8495-8522 |
| | |

| **Vpu:** | **6050-6304** |
|---|---|
| Transmembrane domain | 6050-6146 |
| Cytoplasmic domain | 6147-6304 |
| | |

| **Env (gp160):** | **6228-8786** |
|---|---|
| Signal peptide | 6228-6303 |
| gp120 | 6304-7727 |
| V1 | 6609-6680 |
| V2 | 6781-6812 |
| V3 | 7100-7215 |
| V4 | 7371-7447 |
| V5 | 7575-7608 |
| C1 | 6304-6608 |
| C2 | 6813-7099 |
| C3 | 7216-7370 |
| C4 | 7448-7574 |
| C5 | 7609-7727 |
| CD4 binding | 7482-7508 |
| gp41 | 7728-8786 |
| Fusion peptide | 7722-7775 |
| Oligomerization domain | 7856-7892 |
| N-terminal heptad repeat | 7853-7961 |
| C-terminal heptad repeat | 8105-8213 |
| Immunodominant region | 7956-8009 |
| | |

| **Nef:** | **8788-9411** |
|---|---|
| Myristoylation | 8781-8808 |
| SH3 binding | 8995-9024 |
| Polypurine tract | 9062-9087 |

It will be readily apparent that one of skill in the art can readily align any sequence to that shown in Table A to determine relative locations of any particular HIV gene. For example, using one of the alignment programs described herein (e.g., BLAST), other HIV Type C sequences can be aligned with MJ4 (Table A) and locations of genes determined.

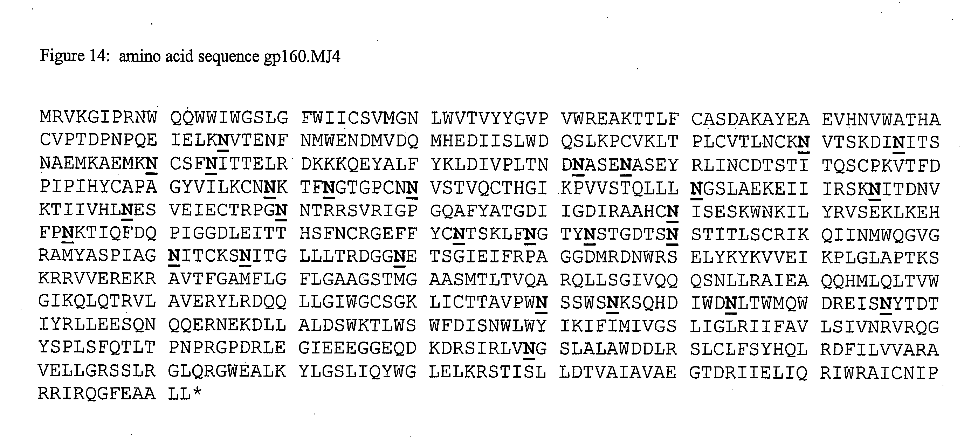

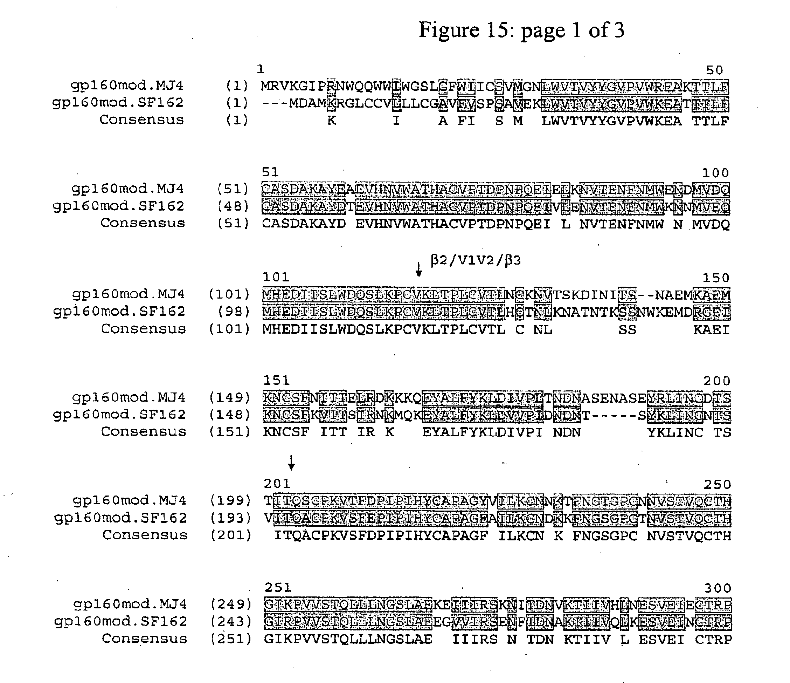

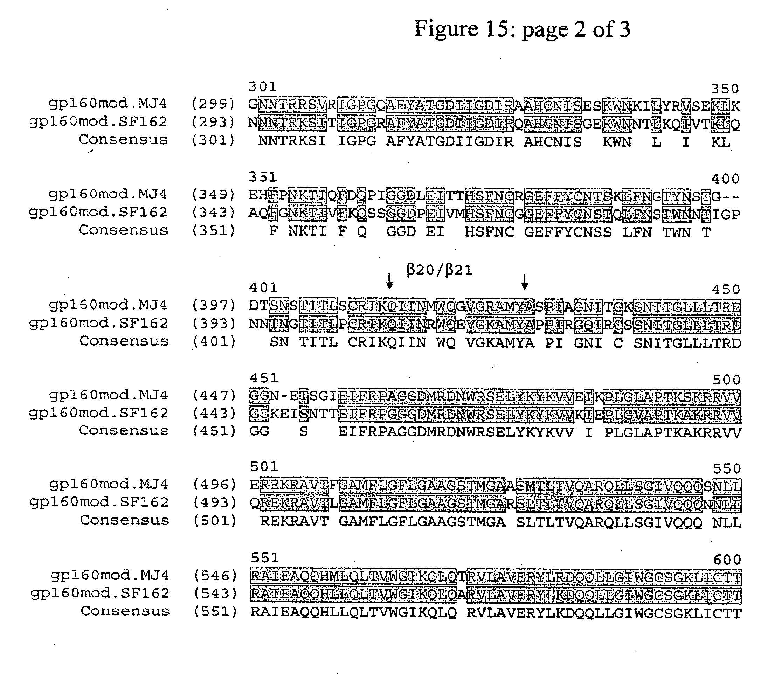

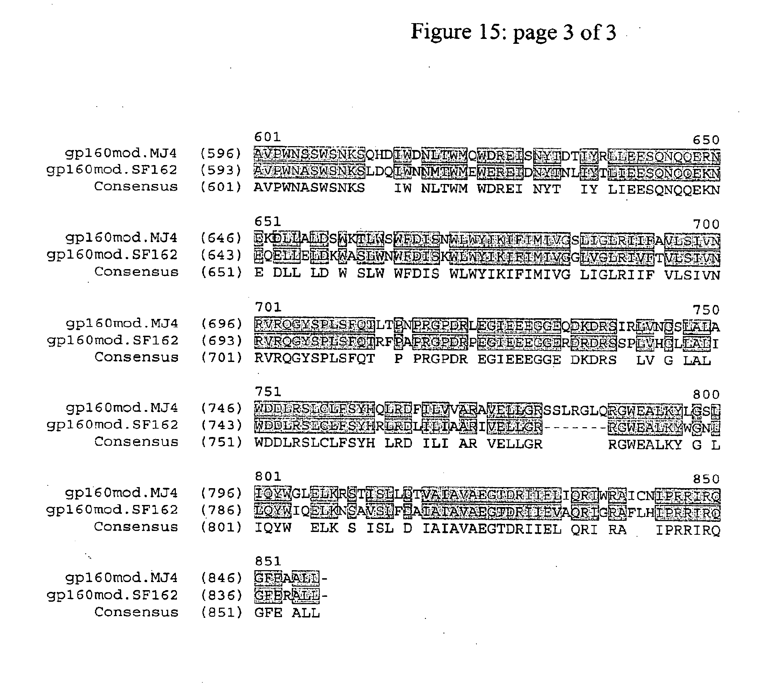

Polypeptide sequences can be similarly aligned. As described in detail in co-owned WO/39303, Env polypeptides (e.g., gp120, gp140 and gp160) include a "bridging sheet" comprised of 4 anti-parallel b-strands (β-2, β-3, β-20 and β-21) that form a β-sheet. Extruding from one pair of the β-strands (β-2, and β-3) are two loops, V1 and V2. The β-2 sheet occurs at approximately amino acid residue 116 (Cys) to amino acid residue 120 (Thr) while β-3 occurs at approximately amino acid residue 200 (Ser) to amino acid residue 203 (Ile), all numbers relative to MJ4. The "V1/V2 region" occurs at approximately amino acid positions 123(Cys) to residue 197 (Cys), relative to MJ4. Extruding from the second pair of β-strands (β-20 and β-21) is a "small-loop" structure, also referred to herein as "the bridging sheet small loop." The locations of both the small loop and bridging sheet small loop can also be determined relative to HXB-2 following the teachings herein and in WO 00/39303. Figure 15 shows an alignment of MJ4 and SF162 gp160s along with a consensus sequence. Arrows indicate the beginning and end of the regions of β2/V1V2/β3 or β20/β21. N-glycosylation sites can be determined (and modified) following the teachings of WO 00/39303. (See, also Figure 14 showing N-glycosylation sites of MJ4 gp160; Pantophlet et al. (2003) J Virol May 15;77(10):5889-901 and Wei et al. (2003) Nature 422(6929):307-312).

### 2.3 Synthetic Polynucleotide Sequences

### 2.3.1 Modification of HIV-1-Type C MJ4 Env Nucleic Acid Coding Sequences

One aspect of the present disclosure is the generation of HIV-1 type C coding sequences, and related sequences, having improved expression and/or immunogenicity relative to the corresponding wild-type MJ4 sequences (Figure 9, SEQ ID NO:8).

Described herein are synthetic Env-encoding polynucleotides and modified Env proteins. Wild-type Env sequences are obtained from the MJ4 molecular clone of HIV-1, type C. (see, for example, Ndung'u et al. (2001) J. Virol. 75:4964-4972). It will be readily apparent from the disclosure herein that polynucleotides encoding fragments of Env gp160 (e.g., gp120, gp41, gp140) can be readily obtained from the larger, full-length sequences disclosed herein. It will also be readily apparent that other modifications can be made, for example deletion of regions such as the V1 and/or V2 region; mutation of the cleavage site and the like (see, Example 1). Exemplary sequences of such modification as shown in SEQ ID NO:1 through 7.

Further, Env sequences obtained from other Type C HIV-1 variants may be manipulated in similar fashion following the teachings of the present specification. Such other variants include, but are not limited to, Env protein encoding sequences obtained from the isolates of HIV-1 Type C, described above.

The codon usage pattern for Env was modified as in WO 00/39303, WO 00/39302 and WO 00/39304 so that the resulting nucleic acid coding sequence was comparable to codon usage found in highly expressed human genes. Experiments performed in support of the present disclosure show that the synthetic Env sequences were capable of higher level of protein production relative to the native Env sequences.

Further modifications of Env include, but are not limited to, generating polynucleotides that encode Env polypeptides having mutations and/or deletions therein. For instance, some or all ofhypervariable regions, V1, V2, V3, V4 and/or V5 can be deleted or modified as described herein, particular V1-V3. V1 and V2 regions may mask CCR5 co-receptor binding sites. (See, *e.g.,* Moulard et al. (2002) Proc. Nat'l Acad Sci 14:9405-9416; Srivastava et al. "Purification and characterization of a soluble trimeric envelope protein containing a partial deletion of the V2 loop derived from SF162," *submitted*). Accordingly, some or all of the variable loop regions are deleted, for example to expose potentially conserved neutralizing epitopes. Further, deglycosylation of N-linked sites are also potential targets for modification inasmuch as a high degree of glycosylation also serves to shield potential neutralizing epitopes on the surface of the protein. Additional optional modifications, used alone or in combination with variable region deletes and/or deglycosylation modification, include modifications (e.g., deletions) to the beta-sheet regions (*e.g.,* as described in WO 00/39303), modifications of the leader sequence (*e.g*., addition of Kozak sequences and/or replacing the modified wild type leader with a native or sequence-modified tpa leader sequence) and/or modifications to protease cleavage sites (*See, e.g.,* Srivastava et al. Srivastava et al. (2003) J Virol. 77(20):11244-59). See, also, Chakrabarti et al. (2002) J. Virol. 76(11):5357-5368 describing a gp140 Delta CFI containing deletions in the cleavage site, fusogenic domain of gp41, and spacing of heptad repeats 1 and 2 of gp41 that retained native antigenic conformational determinants as defined by binding to known monoclonal antibodies or CD4, oligomer formation, and virus neutralization in vitro.
Various combinations of these modifications can be employed to generate synthetic polynucleotide sequences as described herein.
Modification of the Env polypeptide coding sequences results in (1) improved expression relative to the wild-type coding sequences in a number of mammalian cell lines (as well as other types of cell lines, including, but not limited to, insect cells) and/or (2) improved presentation of neutralizing epitopes. Similar Env polypeptide coding sequences can be obtained, modified and tested for improved expression from a variety of isolates.

Synthetic polynucleotide sequences exemplified herein include those depicted in figures 2-8 (Env gp160- and gp140-encoding sequences, modified based MJ4).

HIV polypeptide coding sequences can be obtained from other Type C HIV isolates, see, e.g., Myers et al. Los Alamos Database, Los Alamos National Laboratory, Los Alamos, New Mexico (1992); Myers et al., Human Retroviruses and Aids, 1997, Los Alamos, New Mexico: Los Alamos National Laboratory. Synthetic sequences (and/or vectors containing these sequences) can be generated using such coding sequences as starting material by following the teachings of the present specification (e.g., see Example 1).

Further, the synthetic sequences of the present invention include related polynucleotide sequences having greater than 90%, more preferably greater than 95%, and most preferably greater than 98% sequence identity to particular synthetic polynucleotide sequences. Sequences exhibiting the requisite homology may be generated, for example, by gene shuffling techniques as described for example in U.S. Patent Nos. 6,323,030; 6,444,468; 6,420,175; and 6,413,774. As shown in the following table, Env encoding cassettes may include the following sequences:

| **Region Name** | **Nucleotide number of** **Figure 2** **(SEQ ID NO:1)** |
|---|---|
| Leader sequence | 1-75 |
| Gp120 | 1-1500 |
| Gp41 | 2014-2559 |
| Gp140 | 1-2013 |
| Gp160 | 1-2559 |

### 2.3.2. Further Modification of Sequences Including HIV-1 Env Encoding Sequences

The Type C HIV Env polypeptide-encoding sequences and vectors described herein may also contain one or more further sequences encoding, for example, one or more transgenes. Further sequences (*e.g*., transgenes) useful in the practice of the present invention include, but are not limited to, viral epitopes/antigens {including but not limited to, HCV antigens (e.g., E1, E2; Houghton, M., et al., U.S. Patent No. 5,714,596, issued February 3, 1998; Houghton, M.., et al., U.S. Patent No. 5,712,088, issued January 27, 1998; Houghton, M.., et al., U.S. Patent No. 5,683,864, issued November 4, 1997; Weiner, A.J., et al., U.S. Patent No. 5,728,520, issued March 17, 1998; Weiner, A.J., et al., U.S. Patent No. 5,766,845, issued June 16, 1998; Weiner, A.J., et al., U.S. Patent No. 5,670,152, issued September 23, 1997), HIV antigens (e.g., derived from Gag, tat, rev, nef and/or env).

Further sequences may also be derived from non-viral sources, for instance, sequences encoding tumor antigens, sequences encoding immunomodulatory factors such as cytokines like stem cell factor (SCF), MIP-1I, tumor necrosis factor (TNF), leukemia inhibitory factor (LIF), c-kit ligand, thrombopoietin (TPO) and flt3 ligand, commercially available from several vendors such as, for example, Genzyme (Framingham, MA), Genentech (South San Francisco, CA), Amgen (Thousand Oaks, CA), R&D Systems and Immunex (Seattle, WA). Additional examples of other suitable immunomodulatory molecules for use herein include the following: IL-1 and IL-2 (Karupiah et al. (1990) J. Immunology 144:290-298, Weber et al. (1987) J. Exp. Med. 166:1716-1733, Gansbacher et al. (1990) J. Exp. Med. 172:1217-1224, and U.S. Patent No. 4,738,927); IL-3 and IL-4 (Tepper et al. (1989) Cell 57:503-512, Golumbek et al. (1991) Science 254:713-716, and U.S. Patent No. 5,017,691); IL-5 and IL-6 (Brakenhof et al. (1987) J. Immunol. 139:4116-4121, and International Publication No. WO 90/06370); IL-7 (U.S. Patent No. 4,965,195); IL-8, IL-9, IL-10, IL-11, IL-12, and IL-13 (Cytokine Bulletin, Summer 1994); IL-14 and IL-15; alpha interferon (Finter et al. (1991) Drugs 42:749-765, U.S. Patent Nos. 4,892,743 and 4,966,843, International Publication No. WO 85/02862, Nagata et al. (1980) Nature 284:316-320, Familletti et al. (1981) Methods in Enzymology. 78:387-394, Twu et al. (1989) Proc. Natl. Acad. Sci. USA 86:2046-2050, and Faktor et al. (1990). Oncogene 5:867-872); beta-interferon (Seif et al. (1991) J. Virol. 65:664-671); gamma-interferons (Radford et al. (1991) The American Society of Hepatology 20082015, Watanabe et al. (1989) Proc. Natl. Acad. Sci. USA 86:9456-9460, Gansbacher et al. (1990) Cancer Research 50:7820-7825, Maio et al. (1989) Can. Immunol. Immunother. 30:34-42, and U.S. Patent Nos. 4,762,791 and 4,727,138); G-CSF (U.S. Patent Nous. 4,999,291 and 4,810,643); GM-CSF (International Publication No. WO 85/04188). Sequences encoding muteins of these proteins can also be used (*See, e.g.,* U.S. Patent No. 4,853,332). Nucleic acid sequences encoding the short and long forms of mCSF can be obtained as described in US Patent Nos. 4,847,201 and 4,879,227, respectively. In particular aspects of the invention, retroviral vectors expressing cytokine or immunomodulatory genes can be produced as described herein (for example, employing the packaging cell lines of the present disclosure) and in International Application No. PCT US 94/02951, entitled "Compositions and Methods for Cancer Immunotherapy."

Immunomodulatory factors may also be agonists, antagonists, or ligands for these molecules. For example, soluble forms of receptors can often behave as antagonists for these types of factors, as can mutated forms of the factors themselves.

Nucleic acid molecules that encode the above-described substances, as well as other nucleic acid molecules that are advantageous for use within the present disclosure, may be readily obtained from a variety of sources, including, for example, depositories such as the American Type Culture Collection, or from commercial sources such as British Bio-Technology Limited (Cowley, Oxford England). Representative examples include BBG 12 (containing the GM-CSF gene coding for the mature protein of 127 amino acids), BBG 6 (which contains sequences encoding gamma interferon), A.T.C.C. Deposit No. 39656 (which contains sequences encoding TNF), A.T.C.C. Deposit No. 20663 (which contains sequences encoding alpha-interferon), A.T.C.C. Deposit Nos. 31902, 31902 and 39517 (which contain sequences encoding beta-interferon), A.T.C.C. Deposit No. 67024 (which contains a sequence which encodes Interleukin-1b), A.T.C.C. Deposit Nos. 39405, 39452, 39516, 39626 and 39673 (which contain sequences encoding Interleukin-2), A.T.C.C. Deposit Nos. 59399, 59398, and 67326 (which contain sequences encoding Interleukin-3), A.T.C.C. Deposit No. 57592 (which contains sequences encoding Interleukin-4), A.T.C.C. Deposit Nos. 59394 and 59395 (which contain sequences encoding Interleukin-5), and A.T.C.C. Deposit No. 67153 (which contains sequences encoding Interleukin-6).

Plasmids containing cytokine genes or immunomodulatory genes (International Publication Nos. WO 94/02951 and WO 96/21015) can be digested with appropriate restriction enzymes, and DNA fragments containing the particular gene of interest can be inserted into a gene transfer vector using standard molecular biology techniques. (See, e.g., Sambrook et al., supra., or Ausbel et al. (eds) Current Protocols in Molecular Biology, Greene Publishing and Wiley-Interscience).

Thus, polynucleotide sequences coding for any of the above-described molecules can be obtained using recombinant methods, such as by screening cDNA and genomic libraries from cells expressing the gene, or by deriving the gene from a vector known to include the same. For example, plasmids that contain sequences that encode altered cellular products may be obtained from a depository such as the A.T.C.C., or from commercial sources. Plasmids containing the nucleotide sequences of interest can be digested with appropriate restriction enzymes, and DNA fragments containing the nucleotide sequences can be inserted into a gene transfer vector using standard molecular biology techniques.

Alternatively, cDNA sequences for use with the present invention may be obtained from cells that express or contain the sequences, using standard techniques, such as phenol extraction and PCR of cDNA or genomic DNA. See, e.g., Sambrook et al., supra, for a description of techniques used to obtain and isolate DNA. Briefly, mRNA from a cell that expresses the gene of interest can be reverse transcribed with reverse transcriptase using oligo-dT or random primers. The single stranded cDNA may then be amplified by PCR (see U.S. Patent Nos. 4,683,202, 4,683,195 and 4,800,159, see also PCR Technology: Principles and Applications for DNA Amplification, Erlich (ed.), Stockton Press, 1989)) using oligonucleotide primers complementary to sequences on either side of desired sequences.

The nucleotide sequence of interest can also be produced synthetically, rather than cloned, using a DNA synthesizer (e.g., an Applied Biosystems Model 392 DNA Synthesizer, available from ABI, Foster City, California). The nucleotide sequence can be designed with the appropriate codons for the expression product desired. The complete sequence is assembled from overlapping oligonucleotides prepared by standard methods and assembled into a complete coding sequence. See, e.g., Edge (1981) Nature 292:756; Nambair et al. (1984) Science 223:1299; Jay et al. (1984) J. Biol. Chem. 259:6311.

Various forms of the different embodiments of the invention, described herein, may be combined.

### 2.3.3 Expression of Synthetic Sequences Encoding HIV-1 Subtype C and Related Polypeptides

Synthetic HIV-encoding sequences of the present invention can be cloned into a number of different expression vectors to evaluate levels of expression. The synthetic DNA fragments for HIV polypeptides can be cloned into eukaryotic expression vectors, including, a transient expression vector, CMV-promoter-based mammalian vectors, and a shuttle vector for use in baculovirus expression systems. Corresponding wild-type sequences can also be cloned into the same vectors. Any of the expression constructs (expressing the polypeptides encoded by the polynucleotides described herein) can be used for transiently or stably polypeptide expression, as described in further detail below.

These vectors can then be transfected into a several different cell types, including a variety of mammalian cell lines (293, RD, COS-7, and CHO, cell lines available, for example, from the A.T.C.C.). The cell lines are then cultured under appropriate conditions and the levels of any appropriate polypeptide product can be evaluated in supernatants. For example, gp160, gp140 or gp120 can be used to evaluate Env expression. Further, modified polypeptides can also be used, for example, other Env polypeptides include, but are not limited to, for example, native gp160, oligomeric gp140, monomeric gp120 as well as modified and/or synthetic sequences of these polypeptides. The results of these assays demonstrate that expression of synthetic HIV polypeptide-encoding sequences are significantly higher than corresponding wild-type sequences.

Further, Western Blot analysis can be used to show that cells comprising the synthetic polynucleotides (e.g., expression cassettes comprising these polynucleotides) produce the expected protein at higher per-cell concentrations than cells containing the native sequences. The HIV proteins can be seen in both cell lysates and supernatants (significantly higher in cell supernatants).

Fractionation of the supernatants from mammalian cells transfected as described herein can be used to show that vectors comprising the synthetic sequences described herein provide superior production of HIV proteins.

Efficient expression of these HIV-containing polypeptides in mammalian cell lines provides the following benefits: the polypeptides are free ofbaculovirus contaminants; production by established methods approved by the FDA; increased purity; greater yields (relative to native coding sequences); and a novel method of producing the Subtype C HIV-containing polypeptides in CHO cells which is not feasible in the absence of the increased expression obtained using the constructs of the present invention. Exemplary Mammalian cell lines include, but are not limited to, BHK, VERO, HT1080, 293, 293T, RD, COS-7, CHO, Jurkat, HUT, SUPT, C8166, MOLT4/clone8, MT-2, MT-4, H9, PM1, CEM, and CEMX174, such cell lines are available, for example, from the A.T.C.C.).

Further, synthetic sequences of the present invention can also be introduced into yeast vectors which, in turn, can be transformed into and efficiently expressed by yeast cells (Saccharomyces cerevisea; using vectors as described in Rosenberg, S. and Tekamp-Olson, P., U.S. Patent No. RE35,749, issued, March 17, 1998).

In addition to the mammalian and insect vectors, the synthetic polynucleotides of the present invention can be incorporated into a variety of expression vectors using selected expression control elements. Appropriate vectors and control elements for any given cell type can be selected by one having ordinary skill in the art in view of the teachings of the present specification and information know in the art.

For example, a suitable vector may include control elements operably linked to the desired coding sequence, which allow for the expression of the gene in a selected cell-type. For example, typical promoters for mammalian cell expression include the SV40 early promoter, a CMV promoter such as the CMV immediate early promoter (a CMV promoter can include intron A), RSV, HIV-Ltr, the mouse mammary tumor virus LTR promoter (MMLV-ltr), the adenovirus major late promoter (Ad MLP), and the herpes simplex virus promoter, among others. Other nonviral promoters, such as a promoter derived from the murine metallothionein gene, will also find use for mammalian expression. Typically, transcription termination and polyadenylation sequences will also be present, located 3' to the translation stop codon. Preferably, a sequence for optimization of initiation of translation, located 5' to the coding sequence, is also present. Examples of transcription terminator/polyadenylation signals include those derived from SV40, as described in Sambrook, et al., supra, as well as a bovine growth hormone terminator sequence. Introns, containing splice donor and acceptor sites, may also be designed into the constructs for use with the present invention (Chapman et al., Nuc. Acids Res. (1991) 19:3979-3986).

Enhancer elements may also be used herein to increase expression levels of the mammalian constructs. Examples include the SV40 early gene enhancer, as described in Dijkema et al., EMBO J. (1985) 4:761, the enhancer/promoter derived from the long terminal repeat (LTR) of the Rous Sarcoma Virus, as described in Gorman et al., Proc. Natl. Acad. Sci. USA (1982b) 79:6777 and elements derived from human CMV, as described in Boshart et al., Cell (1985) 41:521, such as elements included in the CMV intron A sequence (Chapman et al., Nuc. Acids Res. (1991) 19:3979-3986).

The desired synthetic polypeptide encoding sequences can be cloned into any number of commercially available vectors to generate expression of the polypeptide in an appropriate host system. These systems include, but are not limited to, the following: baculovirus expression {Reilly, P.R., et al., Baculovirus Expression Vectors: A Laboratory Manual (1992); Beames, et al., Biotechniques 11:378 (1991); Pharmingen; Clontech, Palo Alto, CA)}, vaccinia expression {Earl, P. L., et al., "Expression of proteins in mammalian cells using vaccinia" In Current Protocols in Molecular Biology (F. M. Ausubel, et al. Eds.), Greene Publishing Associates & Wiley Interscience, New York (1991); Moss, B., et al., U.S. Patent Number 5,135,855, issued 4 August 1992}, expression in bacteria {Ausubel, F.M., et al., Current Protocols in Molecular Biology, John Wiley and Sons, Inc., Media PA; Clontech}, expression in yeast {Rosenberg, S. and Tekamp-Olson, P., U.S. Patent No. RE35,749, issued, March 17, 1998 ; Shuster, J.R., U.S. Patent No. 5,629,203, issued May 13, 1997; Gellissen, G., et al., Antonie Van Leeuwenhoek, 62(1-2):79-93 (1992); Romanos, M.A., et al., Yeast 8(6):423-488 (1992); Goeddel, D.V., Methods in Enzymology 185 (1990); Guthrie, C., and G.R. Fink, Methods in Enzymology 194 (1991)}, expression in mammalian cells {Clontech; Gibco-BRL, Ground Island, NY; e.g., Chinese hamster ovary (CHO) cell lines (Haynes, J., et al., Nuc. Acid. Res. 11:687-706 (1983); 1983, Lau, Y.F., et al., Mol. Cell. Biol. 4:1469-1475 (1984); Kaufman, R. J., "Selection and coamplification of heterologous genes in mammalian cells," in Methods in Enzymology, vol. 185, pp537-566. Academic Press, Inc., San Diego CA (1991)}, and expression in plant cells {plant cloning vectors, Clontech Laboratories, Inc., Palo Alto, CA, and Pharmacia LKB Biotechnology, Inc., Pistcataway, NJ; Hood, E., et al., J. Bacteriol. 168:1291-1301 (1986); Nagel, R., et al., FEMS Microbiol. Lett. 67:325 (1990); An, et al., "Binary Vectors", and others in Plant Molecular Biology Manual A3:1-19 (1988); Miki, B.L.A., et al., pp.249-265, and others in Plant DNA Infectious Agents (Hohn, T., et al., eds.) Springer-Verlag, Wien, Austria, (1987); Plant Molecular Biology: Essential Techniques, P.G. Jones and J.M. Sutton, New York, J. Wiley, 1997; Miglani, Gurbachan Dictionary of Plant Genetics and Molecular Biology, New York, Food Products Press, 1998; Henry, R. J., Practical Applications of Plant Molecular Biology, New York, Chapman & Hall, 1997}.

As noted above, the expression vectors typically contain coding sequences and expression control elements that allow expression of the coding regions in a suitable host. The control elements generally include a promoter, translation initiation codon, and translation and transcription termination sequences, and an insertion site for introducing the insert into the vector. Translational control elements have been reviewed by M. Kozak (e.g., Kozak, M., Mamm. Genome 7(8):563-574, 1996; Kozak, M., Biochimie 76(9):815-821, 1994; Kozak, M., J Cell Biol 108(2):229-241, 1989; Kozak, M., and Shatkin, A.J., Methods Enzymol 60:360-375, 1979).

Expression in yeast systems has the advantage of commercial production. Recombinant protein production by vaccinia and CHO cell line have the advantage of being mammalian expression systems. Further, vaccinia virus expression has several advantages including the following: (i) its wide host range; (ii) faithful post-transcriptional modification, processing, folding, transport, secretion, and assembly of recombinant proteins; (iii) high level expression of relatively soluble recombinant proteins; and (iv) a large capacity to accommodate foreign DNA.

The recombinantly expressed polypeptides from synthetic HIV polypeptide-encoding sequences are typically isolated from lysed cells or culture media. Purification can be carried out by methods known in the art including salt fractionation, ion exchange chromatography, gel filtration, size-exclusion chromatography, size-fractionation, and affinity chromatography. Immunoaffinity chromatography can be employed using antibodies generated based on, for example, HIV antigens.

Advantages of expressing the proteins of the present invention using mammalian cells include, but are not limited to, the following: well-established protocols for scale-up production; the ability to produce neutralizing antibodies; cell lines are suitable to meet good manufacturing process (GMP) standards; culture conditions for mammalian cells are known in the art.

In addition, the proteins of the present invention can also be used in conjunction with CD4 proteins, for example in complexes and/or hybrids as described in co-owned International Publication WO 04/037847.

### 2.4 DNA Immunization and Gene Delivery

A variety of HIV polypeptide antigens, particularly Type C HIV antigens, can be used in the practice of the present disclosure. HIV antigens can be included in DNA immunization constructs containing, for example, a synthetic Env sequence (e.g., in a vector such as an expression cassette) fused in-frame to a coding sequence for the polypeptide antigen (synthetic or wild-type), where expression of the construct results in VLPs presenting the antigen of interest.

Other HIV antigens of particular interest to be used in the practice of the present disclosure include Gag, pol, RT, int, tat, rev, nef, vif, vpu, vpr, and other HIV antigens or epitopes derived therefrom. These antigens may be synthetic (as described herein) or wild-type. Further, the packaging cell line may contain only nef, and HIV-1 (also known as HTLV-III, LAV, ARV, etc.), including, but not limited to, antigens (both native and modified) from a variety of isolates including, but not limited to, HIVIIIb, HIVSF2, HIV-1SF162, HIV-1SF170, HIVLAV, HIVLAI, HIVMN, HIV-1CM235" HIV-1US4, other HIV-1 strains from diverse subtypes(e.g., subtypes, A through K, N and O), HIV-2 strains and diverse subtypes (e.g., HIV-2UC1 and HIV-2UC2). See, e.g., Myers, et al., Los Alamos Database, Los Alamos National Laboratory, Los Alamos, New Mexico; Myers, et al., Human Retroviruses and Aids, 1990, Los Alamos, New Mexico: Los Alamos National Laboratory.

To evaluate efficacy, DNA immunization can be performed, for instance as described in Example 4. Animals (*e.g*., mice, rabbits or non-human primates) are immunized with the synthetic Env sequences (*e.g*., expression cassette) and the wild type Env sequences. Immunizations with the polynucleotides will show that the synthetic sequences provide a clear improvement of immunogenicity relative to the native sequences. Also, the second boost immunization will induce a secondary immune response, for example, after approximately two weeks. Further, the results will show increased potency of synthetic Env sequences for induction of neutralizing antibody responses via DNA immunization.

It is readily apparent that the present disclosure can be used to mount an immune response to a wide variety of antigens and hence to treat or prevent a HIV infection, particularly Type C HIV infection.

### 2.4.1 Delivery of the synthetic sequences and vectors of the present invention

Polynucleotide sequences coding for the above-described molecules can be obtained using recombinant methods, such as by screening cDNA and genomic libraries from cells expressing the gene, or by deriving the gene from a vector known to include the same. Furthermore, the desired gene can be isolated directly from cells and tissues containing the same, using standard techniques, such as phenol extraction and PCR of cDNA or genomic DNA. See, e.g., Sambrook et al., supra, for a description of techniques used to obtain and isolate DNA. The gene of interest can also be produced synthetically, rather than cloned. The nucleotide sequence can be designed with the appropriate codons for the particular amino acid sequence desired. In general, one will select preferred codons for the intended host in which the sequence will be expressed. The complete sequence is assembled from overlapping oligonucleotides prepared by standard methods and assembled into a complete coding sequence. See, e.g., Edge, Nature (1981) 292:756; Nambair et al., Science (1984) 223:1299; Jay et al., J. Biol. Chem. (1984) 259:6311; Stemmer, W.P.C., (1995) Gene 164:49-53.

Next, the gene sequence encoding the desired antigen can be inserted into a vector. The vector can also include control elements operably linked to the coding sequence, which allow for the expression of the gene in vivo in the subject species. For example, typical promoters for mammalian cell expression include the SV40 early promoter, a CMV promoter such as the CMV immediate early promoter, the mouse mammary tumor virus LTR promoter, the adenovirus major late promoter (Ad MLP), and the herpes simplex virus promoter, among others. Other nonviral promoters, such as a promoter derived from the murine metallothionein gene, will also find use for mammalian expression. Typically, transcription termination and polyadenylation sequences will also be present, located 3' to the translation stop codon. Preferably, a sequence for optimization of initiation of translation, located 5' to the coding sequence, is also present. Examples of transcription terminator/polyadenylation signals include those derived from SV40, as described in Sambrook et al., supra, as well as a bovine growth hormone terminator sequence.

Enhancer elements may also be used herein to increase expression levels of the mammalian constructs. Examples include the SV40 early gene enhancer, as described in Dijkema et al., EMBO J. (1985) 4:761, the enhancer/promoter derived from the long terminal repeat (LTR) of the Rous Sarcoma Virus, as described in Gorman et al., Proc. Natl. Acad. Sci. USA (1982b) 79:6777 and elements derived from human CMV, as described in Boshart et al., Cell (1985) 41:521, such as elements included in the CMV intron A sequence.

Furthermore, plasmids can be constructed which include a chimeric antigen-coding gene sequences, encoding, e.g., multiple antigens/epitopes of interest, for example derived from more than one viral isolate.

Typically the antigen coding sequences precede or follow the synthetic coding sequence and the chimeric transcription unit will have a single open reading frame encoding both the antigen of interest and the synthetic coding sequences. Alternatively, multi-cistronic cassettes (e.g., bi-cistronic cassettes) can be constructed allowing expression of multiple antigens from a single mRNA using the EMCV IRES, or the like.

Once complete, the constructs are used for nucleic acid immunization using standard gene delivery protocols. Methods for gene delivery are known in the art. See, e.g., U.S. Patent Nos. 5,399,346, 5,580,859, 5,589,466. Genes can be delivered either directly to the vertebrate subject or, alternatively, delivered ex vivo, to cells derived from the subject and the cells reimplanted in the subject.

A number of viral based systems have been developed for gene transfer into mammalian cells. For example, retroviruses provide a convenient platform for gene delivery systems. Selected sequences can be inserted into a vector and packaged in retroviral particles using techniques known in the art. The recombinant virus can then be isolated and delivered to cells of the subject either in vivo or ex vivo. A number of retroviral systems have been described (U.S. Patent No. 5,219,740; Miller and Rosman, BioTechniques (1989) 7:980-990; Miller, A.D., Human Gene Therapy (1990) 1:5-14; Scarpa et al., Virology (1991) 180:849-852; Bums et al., Proc. Natl. Acad. Sci. USA (1993) 90:8033-8037; and Boris-Lawrie and Temin, Cur. Opin. Genet. Develop. (1993) 3:102-109.

A number of adenovirus vectors have also been described. Unlike retroviruses which integrate into the host genome, adenoviruses persist extrachromosomally thus minimizing the risks associated with insertional mutagenesis (Haj-Ahmad and Graham, J. Virol. (1986) 57:267-274; Bett et al., J. Virol. (1993) 67:5911-5921; Mittereder et al., Human Gene Therapy (1994) 5:717-729; Seth et al., J. Virol. (1994) 68:933-940; Barr et al., Gene Therapy (1994) 1:51-58; Berkner, K.L. BioTechniques (1988) 6:616-629; and Rich et al., Human Gene Therapy (1993) 4:461-476).

Additionally, various adeno-associated virus (AAV) vector systems have been developed for gene delivery. AAV vectors can be readily constructed using techniques well known in the art. See, e.g., U.S. Patent Nos. 5,173,414 and 5,139,941; International Publication Nos. WO 92/01070 (published 23 January 1992) and WO 93/03769 (published 4 March 1993); Lebkowski et al., Molec. Cell. Biol. (1988) 8:3988-3996; Vincent et al., Vaccines 90 (1990) (Cold Spring Harbor Laboratory Press); Carter, B.J. Current Opinion in Biotechnology (1992) 3:533-539; Muzyczka, N. Current Topics in Microbiol. and Immunol. (1992) 158:97-129; Kotin, R.M. Human Gene Therapy (1994) 5:793-801; Shelling and Smith, Gene Therapy (1994) 1:165-169; and Zhou et al., J. Exp. Med. (1994) 179:1867-1875.

Another vector system useful for delivering the polynucleotides of the present invention is the enterically administered recombinant poxvirus vaccines described by Small, Jr., P.A., et al. (U.S. Patent No. 5,676,950, issued October 14, 1997).

Additional viral vectors that will find use for delivering the nucleic acid molecules encoding the antigens of interest include those derived from the pox family of viruses, including vaccinia virus and avian poxvirus. By way of example, vaccinia virus recombinants expressing the genes can be constructed as follows. The DNA encoding the particular synthetic HIV subtype C polypeptide coding sequence is first inserted into an appropriate vector so that it is adjacent to a vaccinia promoter and flanking vaccinia DNA sequences, such as the sequence encoding thymidine kinase (TK). This vector is then used to transfect cells that are simultaneously infected with vaccinia. Homologous recombination serves to insert the vaccinia promoter plus the gene encoding the coding sequences of interest into the viral genome. The resulting TK-recombinant can be selected by culturing the cells in the presence of 5-bromodeoxyuridine and picking viral plaques resistant thereto.

Alternatively, avipoxviruses, such as the fowlpox and canarypox viruses, can also be used to deliver the genes. Recombinant avipox viruses, expressing immunogens from mammalian pathogens, are known to confer protective immunity when administered to non-avian species. The use of an avipox vector is particularly desirable in human and other mammalian species since members of the avipox genus can only productively replicate in susceptible avian species and therefore are not infective in mammalian cells. Methods for producing recombinant avipoxviruses are known in the art and employ genetic recombination, as described above with respect to the production of vaccinia viruses. See, e.g., WO 91/12882; WO 89/03429; and WO 92/03545.

Molecular conjugate vectors, such as the adenovirus chimeric vectors described in Michael et al., J. Biol. Chem. (1993) 268:6866-6869 and Wagner et al., Proc. Natl. Acad. Sci. USA (1992) 89:6099-6103, can also be used for gene delivery.

Members of the Alphavirus genus, such as, but not limited to, vectors derived from the Sindbis, Semliki Forest, and Venezuelan Equine Encephalitis viruses, will also find use as viral vectors for delivering the polynucleotides of the present invention (for example, a synthetic Env-polypeptide encoding expression cassette). For a description of Sindbis-virus derived vectors useful for the practice of the instant methods, see, Dubensky et al., J. Virol. (1996) 70:508-519; and International Publication Nos. WO 95/07995 and WO 96/17072; as well as, Dubensky, Jr., T.W., et al., U.S. Patent No. 5,843,723, issued December 1, 1998, and Dubensky, Jr., T.W., U.S. Patent No. 5,789,245, issued August 4, 1998.

A vaccinia based infection/transfection system can be conveniently used to provide for inducible, transient expression of the coding sequences of interest in a host cell. In this system, cells are first infected in vitro with a vaccinia virus recombinant that encodes the bacteriophage T7 RNA polymerase. This polymerase displays exquisite specificity in that it only transcribes templates bearing T7 promoters. Following infection, cells are transfected with the polynucleotide of interest, driven by a T7 promoter. The polymerase expressed in the cytoplasm from the vaccinia virus recombinant transcribes the transfected DNA into RNA that is then translated into protein by the host translational machinery. The method provides for high level, transient, cytoplasmic production of large quantities of RNA and its translation products. See, e.g., Elroy-Stein and Moss, Proc. Natl. Acad. Sci. USA (1990) 87:6743-6747; Fuerst et al., Proc. Natl. Acad. Sci. USA (1986) 83:8122-8126.

As an alternative approach to infection with vaccinia or avipox virus recombinants, or to the delivery of genes using other viral vectors, an amplification system can be used that will lead to high level expression following introduction into host cells. Specifically, a T7 RNA polymerase promoter preceding the coding region for T7 RNA polymerase can be engineered. Translation of RNA derived from this template will generate T7 RNA polymerase that in turn will transcribe more template. Concomitantly, there will be a cDNA whose expression is under the control of the T7 promoter. Thus, some of the T7 RNA polymerase generated from translation of the amplification template RNA will lead to transcription of the desired gene. Because some T7 RNA polymerase is required to initiate the amplification, T7 RNA polymerase can be introduced into cells along with the template(s) to prime the transcription reaction. The polymerase can be introduced as a protein or on a plasmid encoding the RNA polymerase. For a further discussion of T7 systems and their use for transforming cells, see, e.g., International Publication No. WO 94/26911; Studier and Moffatt, J. Mol. Biol. (1986) 189:113-130; Deng and Wolff, Gene (1994) 143:245-249; Gao et al., Biochem. Biophys. Res. Commun. (1994) 200:1201-1206; Gao and Huang, Nuc. Acids Res. (1993) 21:2867-2872; Chen et al., Nuc. Acids Res. (1994) 22:2114-2120; and U.S. Patent No. 5,135,855.

Synthetic sequences of interest can also be delivered without a viral vector. For example, the synthetic sequences (or expression cassettes) can be packaged in liposomes prior to delivery to the subject or to cells derived therefrom. Lipid encapsulation is generally accomplished using liposomes that are able to stably bind or entrap and retain nucleic acid. The ratio of condensed DNA to lipid preparation can vary but will generally be around 1:1 (mg DNA:micromoles lipid), or more of lipid. For a review of the use of liposomes as carriers for delivery of nucleic acids, see, Hug and Sleight, Biochim. Biophys. Acta. (1991) 1097:1-17; Straubinger et al., in Methods of Enzymology (1983), Vol.101, pp. 512-527.

Liposomal preparations for use in the present invention include cationic (positively charged), anionic (negatively charged) and neutral preparations, with cationic liposomes particularly preferred. Cationic liposomes have been shown to mediate intracellular delivery of plasmid DNA (Felgner et al., Proc. Natl. Acad. Sci. USA (1987) 84:7413-7416); mRNA (Malone et al., Proc. Natl. Acad. Sci. USA (1989) 86:6077-6081); and purified transcription factors (Debs et al., J. Biol. Chem. (1990) 265:10189-10192), in functional form.

Cationic liposomes are readily available. For example, N[1-2,3-dioleyloxy)propyl]-N,N,N-triethylammonium (DOTMA) liposomes are available under the trademark Lipofectin, from GIBCO BRL, Grand Island, NY. (See, also, Felgner et al., Proc. Natl. Acad. Sci. USA (1987) 84:7413-7416). Other commercially available lipids include (DDAB/DOPE) and DOTAP/DOPE (Boerhinger). Other cationic liposomes can be prepared from readily available materials using techniques well known in the art. See, e.g., Szoka et al., Proc. Natl. Acad. Sci. USA (1978) 75:4194-4198; PCT Publication No. WO 90/11092 for a description of the synthesis of DOTAP (1,2-bis(oleoyloxy)-3-(trimethylammonio)propane) liposomes.

Similarly, anionic and neutral liposomes are readily available, such as, from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

The liposomes can comprise multilammelar vesicles (MLVs), small unilamellar vesicles (SUVs), or large unilamellar vesicles (LUVs). The various liposome-nucleic acid complexes are prepared using methods known in the art. See, e.g., Straubinger et al., in METHODS OF IMMUNOLOGY (1983), Vol. 101, pp. 512-527; Szoka et al., Proc. Natl. Acad. Sci. USA (1978) 75:4194-4198; Papahadjopoulos et al., Biochim. Biophys. Acta (1975) 394:483; Wilson et al., Cell (1979) 17:77); Deamer and Bangham, Biochim. Biophys. Acta (1976) 443:629; Ostro et al., Biochem. Biophys. Res. Commun. (1977) 76:836; Fraley et al., Proc. Natl. Acad. Sci. USA (1979) 76:3348); Enoch and Strittmatter, Proc. Natl. Acad. Sci. USA (1979) 76:145); Fraley et al., J. Biol. Chem. (1980) 255:10431; Szoka and Papahadjopoulos, Proc. Natl. Acad. Sci. USA (1978) 75:145; and Schaefer-Ridder et al., Science (1982) 215:166.

The DNA and/or protein antigen(s) can also be delivered in cochleate lipid compositions similar to those described by Papahadjopoulos et al., Biochem. Biophys. Acta. (1975) 394:483-491. See, also, U.S. Patent Nos. 4,663,161 and 4,871,488.

The synthetic sequences (and/or expression cassettes) of interest may also be encapsulated, adsorbed to, or associated with, particulate carriers. Such carriers present multiple copies of a selected antigen to the immune system and promote trapping and retention of antigens in local lymph nodes. The particles can be phagocytosed by macrophages and can enhance antigen presentation through cytokine release. Examples of particulate carriers include those derived from polymethyl methacrylate polymers, as well as microparticles derived from poly(lactides) and poly(lactide-co-glycolides), known as PLG. See, e.g., Jeffery et al., Pharm. Res. (1993) 10:362-368; McGee JP, et al., J Microencapsul. 14(2):197-210, 1997; O'Hagan DT, et al., Vaccine 11(2):149-54, 1993. Suitable microparticles may also be manufactured in the presence of charged detergents, such as anionic or cationic detergents, to yield microparticles with a surface having a net negative or a net positive charge. For example, microparticles manufactured with anionic detergents, such as hexadecyltrimethylammonium bromide (CTAB), i.e. CTAB-PLG microparticles, adsorb negatively charged macromolecules, such as DNA. (see, e.g., Int'l Application Number PCT/US99/17308).

Furthermore, other particulate systems and polymers can be used for the in vivo or ex vivo delivery of the gene of interest. For example, polymers such as polylysine, polyarginine, polyornithine, spermine, spermidine, as well as conjugates of these molecules, are useful for transferring a nucleic acid of interest. Similarly, DEAE dextran-mediated transfection, calcium phosphate precipitation or precipitation using other insoluble inorganic salts, such as strontium phosphate, aluminum silicates including bentonite and kaolin, chromic oxide, magnesium silicate, talc, and the like, will find use with the present methods. See, e.g., Felgner, P.L., Advanced Drug Delivery Reviews (1990) 5:163-187, for a review of delivery systems useful for gene transfer. Peptoids (Zuckerman, R.N., et al., U.S. Patent No. 5,831,005, issued November 3, 1998) may also be used for delivery of a construct of the present invention.

Additionally, biolistic delivery systems employing particulate carriers such as gold and tungsten, are especially useful for delivering synthetic sequences and vectors of the present invention. The particles are coated with the synthetic sequences (and/or expression cassette(s)) to be delivered and accelerated to high velocity, generally under a reduced atmosphere, using a gun powder discharge from a "gene gun." For a description of such techniques, and apparatuses useful therefore, see, e.g., U.S. Patent Nos. 4,945,050; 5,036,006; 5,100,792; 5,179,022; 5,371,015; and 5,478,744. Also, needle-less injection systems can be used (Davis, H.L., et al, Vaccine 12:1503-1509, 1994; Bioject, Inc., Portland, OR). Direct delivery of compositions comprising the synthetic sequences described herein *in vivo* will generally be accomplished with or without viral vectors, as described above, by injection using either a conventional syringe or a gene gun, such as the Accell® gene delivery system (PowderJect Technologies, Inc., Oxford, England). The constructs can be injected either subcutaneously, epidermally, intradermally, intramucosally such as nasally, rectally and vaginally, intraperitoneally, intravenously, orally or intramuscularly. Delivery of DNA into cells of the epidermis is particularly preferred as this mode of administration provides access to skin-associated lymphoid cells and provides for a transient presence of DNA in the recipient. Other modes of administration include oral and pulmonary administration, suppositories, needle-less injection, transcutaneous and transdermal applications.

The sequences described herein may also be administered using *in vivo* electroporation techniques. Efficient in vivo expression of plasmid encoded genes by electrical permeabilization (electroporation) has been described (see, *e.g.*, Zucchelli et al. (2000) J. Virol. 74:11598-11607; Banga et al. (1998) Trends Biotechnol. 10:408-412; Heller et al. (1996) Febs Lett. 389:225-228; Mathiesen et al. (1999) Gene Ther. 4:508-514; Mir et al. (1999) Proc. Nat'l Acad Sci. USA 8:4262-4267; Nishi et al. (1996) Cancer Res. 5:1050-1055*).*

Recombinant vectors carrying a synthetic sequences of the present invention are typically formulated into compositions for delivery to the vertebrate subject. These compositions may either be prophylactic (to prevent infection) or therapeutic (to treat disease after infection) and may include one or more of the following molecules: polynucleotides, polypeptides, other small molecules and/or other macromolecules. The compositions will comprise a "therapeutically effective amount" of the gene of interest such that an amount of the antigen can be produced in vivo so that an immune response is generated in the individual to which it is administered. The exact amount necessary will vary depending on the subject being treated; the age and general condition of the subject to be treated; the capacity of the subject's immune system to synthesize antibodies; the degree of protection desired; the severity of the condition being treated; the particular antigen selected and its mode of administration, among other factors. An appropriate effective amount can be readily determined by one of skill in the art. Thus, a "therapeutically effective amount" will fall in a relatively broad range that can be determined through routine trials.

The compositions will generally include one or more "pharmaceutically acceptable excipients or vehicles" such as water, saline, glycerol, polyethyleneglycol, hyaluronic acid, ethanol, etc. Additionally, auxiliary substances, such as wetting or emulsifying agents, pH buffering substances, and the like, may be present in such vehicles. Certain facilitators of nucleic acid uptake and/or expression can also be included in the compositions or coadministered, such as, but not limited to, bupivacaine, cardiotoxin and sucrose.

A carrier is optionally present which is a molecule that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Examples of particulate carriers include those derived from polymethyl methacrylate polymers, as well as microparticles derived from poly(lactides) and poly(lactide-co-glycolides), known as PLG. See, e.g., Jeffery et al., Pharm. Res. (1993) 10:362-368; McGee JP, et al., J Microencapsul. 14(2):197-210, 1997; O'Hagan DT, et al., Vaccine 11(2):149-54, 1993. Such carriers are well known to those of ordinary skill in the art. Additionally, these carriers may function as immunostimulating agents ("adjuvants"). Furthermore, the antigen may be conjugated to a bacterial toxoid, such as toxoid from diphtheria, tetanus, cholera, etc., as well as toxins derived from E. coli.

Adjuvants may also be used to enhance the effectiveness of the compositions. Such adjuvants include, but are not limited to: (1) aluminum salts (alum), such as aluminum hydroxide, aluminum phosphate, aluminum sulfate, etc.; (2) oil-in-water emulsion formulations (with or without other specific immunostimulating agents such as muramyl peptides (see below) or bacterial cell wall components), such as for example (a) MF59 (International Publication No. WO 90/14837), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (3) saponin adjuvants, such as Stimulon™ (Cambridge Bioscience, Worcester, MA) may be used or particle generated therefrom such as ISCOMs (immunostimulating complexes); (4) Complete Freunds Adjuvant (CFA) and Incomplete Freunds Adjuvant (IFA); (5) cytokines, such as interleulcins (IL-1, IL-2, etc.), macrophage colony stimulating factor (M-CSF), tumor necrosis factor (TNF), etc.; (6) oligonucleotides or polymeric molecules encoding immunostimulatory CpG motifs (Davis, H.L., et al., J. Immunology 160:870-876, 1998; Sato, Y. et al., Science 273:352-354, 1996) or complexes of antigens/oligonucleotides {Polymeric molecules include double and single stranded RNA and DNA, and backbone modifications thereof, for example, methylphosphonate linkages; or (7) detoxified mutants of a bacterial ADP-ribosylating toxin such as a cholera toxin (CT), a pertussis toxin (PT), or an E. coli heat-labile toxin (LT), particularly LT-K63 (where lysine is substituted for the wild-type amino acid at position 63) LT-R72 (where arginine is substituted for the wild-type amino acid at position 72), CT-S109 (where serine is substituted for the wild-type amino acid at position 109), and PT-K9/G129 (where lysine is substituted for the wild-type amino acid at position 9 and glycine substituted at position 129) (see, e.g., International Publication Nos. W093/13202 and W092/19265); and (8) other substances that act as immunostimulating agents to enhance the effectiveness of the composition. Further, such polymeric molecules include alternative polymer backbone structures such as, but not limited to, polyvinyl backbones (Pitha, Biochem Biophys Acta, 204:39, 1970a; Pitha, Biopolymers, 9:965, 1970b), and morpholino backbones (Summerton, J., et al., U.S. Patent No. 5,142,047, issued 08/25/92; Summerton, J., et al., U.S. Patent No. 5,185,444 issued 02/09/93). A variety of other charged and uncharged polynucleotide analogs have been reported. Numerous backbone modifications are known in the art, including, but not limited to, uncharged linkages (e.g., methyl phosphonates, phosphotriesters, phosphoamidates, and carbamates) and charged linkages (e.g., phosphorothioates and phosphorodithioates).}; and (7) other substances that act as immunostimulating agents to enhance the effectiveness of the VLP immune-stimulating (or vaccine) composition. Alum, CpG oligonucleotides, and MF59 are preferred.

Muramyl peptides include, but are not limited to, N-acetyl-muramyl-L-threonyl-D-isoglutamine (thr-MDP), N-acteyl-normuramyl-L-alanyl-D-isogluatme (nor-MDP), N-acetylmuramyl-L-alanyl-D-isogluatminyl-L-alanine-2-(1'-2'-dipalmitoyl-sn-glycero-3-huydroxyphosphoryloxy)-ethylamine (MTP-PE), etc.

Once formulated, the compositions of the invention can be administered directly to the subject (e.g., using one or more of the methods described above) or, alternatively, delivered ex vivo, to cells derived from the subject, using methods such as those described above. For example, methods for the ex vivo delivery and reimplantation of transformed cells into a subject are known in the art and can include, e.g., dextran-mediated transfection, calcium phosphate precipitation, polybrene mediated transfection, lipofectamine and LT-1 mediated transfection, protoplast fusion, electroporation, encapsulation of the polynucleotide(s) (with or without the corresponding antigen) in liposomes, and direct microinjection of the DNA into nuclei.

The amount of DNA administered to the subject may vary depending on the antigens and/or delivery protocol. Thus, in certain embodiments, the amount of DNA used per administration (e.g., immunization) may vary from nanogram to microgram to milligram amounts of DNA, for example, as described in the Examples where the dose given is between about 2 nanograms to 20 micrograms or between about 2 nanograms and 10 milligrams. Further, as described below and shown in the Examples, multiple administrations of DNA (and/or protein) are contemplated.

Dosage treatment may be a single dose schedule or a multiple dose schedule. Administration of nucleic acids may also be combined with administration of peptides or other substances.

### 2.4.2 Ex vivo Delivery

In one embodiment, T cells, and related cell types (including but not limited to antigen presenting cells, such as, macrophage, monocytes, lymphoid cells, dendritic cells, B-cells, T-cells, stem cells, and progenitor cells thereof), can be used for ex vivo delivery of the synthetic sequences of the present invention. T cells can be isolated from peripheral blood lymphocytes (PBLs) by a variety of procedures known to those skilled in the art. For example, T cell populations can be "enriched" from a population of PBLs through the removal of accessory and B cells. In particular, T cell enrichment can be accomplished by the elimination of non-T cells using anti-MHC class II monoclonal antibodies. Similarly, other antibodies can be used to deplete specific populations of non-T cells. For example, anti-Ig antibody molecules can be used to deplete B cells and anti-MacI antibody molecules can be used to deplete macrophages.

T cells can be further fractionated into a number of different subpopulations by techniques known to those skilled in the art. Two major subpopulations can be isolated based on their differential expression of the cell surface markers CD4 and CD8. For example, following the enrichment of T cells as described above, CD4+ cells can be enriched using antibodies specific for CD4 (see Coligan et al., supra). The antibodies may be coupled to a solid support such as magnetic beads. Conversely, CD8+ cells can be enriched through the use of antibodies specific for CD4 (to remove CD4+ cells), or can be isolated by the use of CD8 antibodies coupled to a solid support. CD4 lymphocytes from HIV-1 infected patients can be expanded ex vivo, before or after transduction as described by Wilson et. al. (1995) J. Infect. Dis. 172:88.

Following purification of T cells, a variety of methods of genetic modification known to those skilled in the art can be performed using non-viral or viral-based gene transfer vectors constructed as described herein. For example, one such approach involves transduction of the purified T cell population with vector-containing supernatant of cultures derived from vector producing cells. A second approach involves co-cultivation of an irradiated monolayer of vector-producing cells with the purified T cells. A third approach involves a similar co-cultivation approach; however, the purified T cells are pre-stimulated with various cytokines and cultured 48 hours prior to the co-cultivation with the irradiated vector producing cells. Pre-stimulation prior to such transduction increases effective gene transfer (Nolta et al. (1992) Exp. Hematol. 20:1065). Stimulation of these cultures to proliferate also provides increased cell populations for re-infusion into the patient. Subsequent to co-cultivation, T cells are collected from the vector producing cell monolayer, expanded, and frozen in liquid nitrogen.

Gene transfer vectors, containing one or more synthetic sequences of the present invention (associated with appropriate control elements for delivery to the isolated T cells) can be assembled using known methods.

Selectable markers can also be used in the construction of gene transfer vectors. For example, a marker can be used which imparts to a mammalian cell transduced with the gene transfer vector resistance to a cytotoxic agent. The cytotoxic agent can be, but is not limited to, neomycin, aminoglycoside, tetracycline, chloramphenicol, sulfonamide, actinomycin, netropsin, distamycin A, anthracycline, or pyrazinamide. For example, neomycin phosphotransferase II imparts resistance to the neomycin analogue geneticin (G418).

The T cells can also be maintained in a medium containing at least one type of growth factor prior to being selected. A variety of growth factors are known in the art that sustain the growth of a particular cell type. Examples of such growth factors are cytokine mitogens such as rIL-2, IL-10, IL-12, and IL-15, which promote growth and activation of lymphocytes. Certain types of cells are stimulated by other growth factors such as hormones, including human chorionic gonadotropin (hCG) and human growth hormone. The selection of an appropriate growth factor for a particular cell population is readily accomplished by one of skill in the art.

For example, white blood cells such as differentiated progenitor and stem cells are stimulated by a variety of growth factors. More particularly, IL-3, IL-4, IL-5, IL-6, IL-9, GM-CSF, M-CSF, and G-CSF, produced by activated TH and activated macrophages, stimulate myeloid stem cells, which then differentiate into pluripotent stem cells, granulocyte-monocyte progenitors, eosinophil progenitors, basophil progenitors, megakaryocytes, and erythroid progenitors. Differentiation is modulated by growth factors such as GM-CSF, IL-3, IL-6, IL-11, and EPO.

Pluripotent stem cells then differentiate into lymphoid stem cells, bone marrow stromal cells, T cell progenitors, B cell progenitors, thymocytes, TH Cells, TC cells, and B cells. This differentiation is modulated by growth factors such as IL-3, IL-4, IL-6, IL-7, GM-CSF, M-CSF, G-CSF, IL-2, and IL-5.

Granulocyte-monocyte progenitors differentiate to monocytes, macrophages, and neutrophils. Such differentiation is modulated by the growth factors GM-CSF, M-CSF, and IL-8. Eosinophil progenitors differentiate into eosinophils. This process is modulated by GM-CSF and EL-5.

The differentiation of basophil progenitors into mast cells and basophils is modulated by GM-CSF, IL-4, and IL-9. Megakaryocytes produce platelets in response to GM-CSF, EPO, and IL-6. Erythroid progenitor cells differentiate into red blood cells in response to EPO.

Thus, during activation by the CD3-binding agent, T cells can also be contacted with a mitogen, for example a cytokine such as IL-2. In particularly preferred embodiments, the IL-2 is added to the population of T cells at a concentration of about 50 to 100 µg/ml. Activation with the CD3-binding agent can be carried out for 2 to 4 days.

Once suitably activated, the T cells are genetically modified by contacting the same with a suitable gene transfer vector under conditions that allow for transfection of the vectors into the T cells. Genetic modification is carried out when the cell density of the T cell population is between about 0.1 x 106 and 5 x 106, preferably between about 0.5 x 10⁶ and 2 x 10⁶. A number of suitable viral and nonviral-based gene transfer vectors have been described for use herein.

After transduction, transduced cells are selected away from non-transduced cells using know techniques. For example, if the gene transfer vector used in the transduction includes a selectable marker that confers resistance to a cytotoxic agent, the cells can be contacted with the appropriate cytotoxic agent, whereby non-transduced cells can be negatively selected away from the transduced cells. If the selectable marker is a cell surface marker, the cells can be contacted with a binding agent specific for the particular cell surface marker, whereby the transduced cells can be positively selected away from the population. The selection step can also entail fluorescence-activated cell sorting (FACS) techniques, such as where FACS is used to select cells from the population containing a particular surface marker, or the selection step can entail the use of magnetically responsive particles as retrievable supports for target cell capture and/or background removal.

More particularly, positive selection of the transduced cells can be performed using a FACS cell sorter (e.g. a FACSVantage™ Cell Sorter, Becton Dickinson Immunocytometry Systems, San Jose, CA) to sort and collect transduced cells expressing a selectable cell surface marker. Following transduction, the cells are stained with fluorescent-labeled antibody molecules directed against the particular cell surface marker. The amount of bound antibody on each cell can be measured by passing droplets containing the cells through the cell sorter. By imparting an electromagnetic charge to droplets containing the stained cells, the transduced cells can be separated from other cells. The positively selected cells are then harvested in sterile collection vessels. These cell sorting procedures are described in detail, for example, in the FACSVantage™ Training Manual, with particular reference to sections 3-11 to 3-28 and 10-1 to 10-17.

Positive selection of the transduced cells can also be performed using magnetic separation of cells based on expression or a particular cell surface marker. In such separation techniques, cells to be positively selected are first contacted with specific binding agent (e.g., an antibody or reagent the interacts specifically with the cell surface marker). The cells are then contacted with retrievable particles (e.g., magnetically responsive particles) that are coupled with a reagent that binds the specific binding agent (that has bound to the positive cells). The cell-binding agent-particle complex can then be physically separated from non-labeled cells, for example using a magnetic field. When using magnetically responsive particles, the labeled cells can be retained in a container using a magnetic filed while the negative cells are removed. These and similar separation procedures are known to those of ordinary skill in the art.

Expression of the vector in the selected transduced cells can be assessed by a number of assays known to those skilled in the art. For example, Western blot or Northern analysis can be employed depending on the nature of the inserted nucleotide sequence of interest. Once expression has been established and the transformed T cells have been tested for the presence of the selected synthetic sequence, they are ready for infusion into a patient via the peripheral blood stream.

The disclosure includes a kit for genetic modification of an ex vivo population of primary mammalian cells. The kit typically contains a gene transfer vector coding for at least one selectable marker and at least one synthetic sequence (*e.g*., expression cassette) contained in one or more containers, ancillary reagents or hardware, and instructions for use of the kit.

### 2.4.3 Further Delivery regimes

Any of the polynucleotides (*e.g*., expression cassettes) or polypeptides described herein (delivered by any of the methods described above) can also be used in combination with other DNA delivery systems and/or protein delivery systems. Non-limiting examples include co-administration of these molecules, for example, in prime-boost methods where one or more molecules are delivered in a "priming" step and, subsequently, one or more molecules are delivered in a "boosting" step. For example, priming immunizations with DNA vectors expressing the viral envelope followed by booster immunizations with soluble proteins appear to generate anti-envelope antibodies that have higher neutralizing activities than antibodies generated by immunization with soluble proteins (Richmond (1998) J Virol. 72:9092-100).

In certain embodiments, the delivery of one or more nucleic acid-containing compositions and is followed by delivery of one or more nucleic acid-containing compositions and/or one or more polypeptide-containing compositions (e.g., polypeptides comprising HIV antigens). In other embodiments, multiple nucleic acid "primes" (of the same or different nucleic acid molecules) can be followed by multiple polypeptide "boosts" (of the same or different polypeptides). Other examples include multiple nucleic acid administrations and multiple polypeptide administrations. In any of these embodiments, the co-administered compositions may be derived from HIV strain MJ4 or from one or more different strains.

In any method involving co-administration, the various compositions can be delivered in any order. Thus, in embodiments including delivery of multiple different compositions or molecules, the nucleic acids need not be all delivered before the polypeptides. For example, the priming step may include delivery of one or more polypeptides and the boosting comprises delivery of one or more nucleic acids and/or one more polypeptides. Multiple polypeptide administrations can be followed by multiple nucleic acid administrations or polypeptide and nucleic acid administrations can be performed in any order. In any of the disclosure described herein, the nucleic acid molecules can encode all, some or none of the polypeptides. Thus, one or more or the nucleic acid molecules (e.g., expression cassettes) described herein and/or one or more of the polypeptides described herein can be co-administered in any order and via any administration routes. Therefore, any combination of polynucleotides and/or polypeptides described herein can be used to generate elicit an immune reaction.

### EXPERIMENTAL

Below are examples of specific embodiments for carrying out the present invention. The examples are offered for illustrative purposes only, and are not intended to limit the scope of the present invention in any way.

Efforts have been made to ensure accuracy with respect to numbers used (e.g., amounts, temperatures, etc.), but some experimental error and deviation should, of course, be allowed for.

### EXAMPLE 1

### GENERATION OF SYNTHETIC SEQUENCES

The Env coding sequences were selected from Type C strain MJ4. These sequences were manipulated to maximize expression of their gene products.

First, the HIV-1 codon usage pattern was modified so that the resulting nucleic acid coding sequence was comparable to codon usage found in highly expressed human genes. The HIV codon usage reflects a high content of the nucleotides A or T of the codon-triplet. The effect of the HIV-1 codon usage is a high AT content in the DNA sequence that results in a decreased translation ability and instability of the mRNA. In comparison, highly expressed human codons prefer the nucleotides G or C. The coding sequences were modified to be comparable to codon usage found in highly expressed human genes.

Certain Env-encoding sequences were also modified such that V1 and/or V2 were deleted; to modify the leader sequence to a tpa leader sequence and/or to mutate the protease cleavage site.

The synthetic coding sequences are assembled by methods known in the art, for example by companies such as the Midland Certified Reagent Company (Midland, Texas) or RetroGen (San Diego, California).

The synthetic DNA fragments for Env are cloned into the following eukaryotic expression vectors: pCMVlink or pCMVKm2. For a description of construction of these vectors, see, for example, WO 00/39302. Exemplary synthetic sequences are shown in Figures 2-8 (Table B below).

**Table B: Exemplary sequences encoding modified MJ4 Env proteins**

| Name | Description |
|---|---|
| gp160mod.MJ4 (SEQ ID NO:1) (Fig.2) | synthetic sequence of Env gp160 derived from wild-type MJ4 |
| gp160mod.MJ4.dV2 (SEQ ID NO:2) (Fig.3) | synthetic sequence of Env gp160, including V2 deletion derived from wild-type MJ4 |
| gp160mod.MJ4.dV1V2 (SEQ ID NO:3) (Fig.4) | synthetic sequence of Env gp160, including V1/V2 deletion derived from wild-type MJ4 |
| gp160mod.MJ4.tpa (SEQ ID NO:4) (Fig.5) | synthetic sequence of Env gp160 derived from wild-type MJ4 with modified leader sequences |
| gp160mod.MJ4.dV2.tpa (SEQ ID NO:5) Fig.6) | synthetic sequence of Env gp160 derived from wild-type MJ4, including V2 delete and modified leader sequences |
| gp1 60mod.MJ4.dV1V2. tpa (SEQ ID NO:6) (Fig.7) | synthetic sequence of Env gp160 derived from wild-type MJ4, including V1/V2 delete and modified leader sequences |
| gp140mod.MJ4.dV2.mut7. tpa (SEQ ID NO:7) (Fig.8) | synthetic sequence of Env gp140 derived from wild-type MJ4, including V2 delete, modified leader sequences and mutated protease cleavage site |

The common sequence region (CSR) of HIV-1 Env is located in the C4 sequence of Env. It is a conserved stretch of approximately 42 amino acids. The position in the wild type and synthetic MJ4-based Env proteins is from approximately amino acid residue 407 to 449 and spans a region from 1222 to 1347 (Fig.9) for the Env DNA-sequence. Percent identity to this sequence can be determined, for example, using the Smith-Waterman search algorithm (Time Logic, Incline Village, NV), with the following exemplary parameters: weight matrix = nuc4x4hb; gap opening penalty = 20, gap extension penalty = 5. Percent identity to this sequence can be determined, for example, using the Smith-Waterman search algorithm (Time Logic, Incline Village, NV), with the following exemplary parameters: weight matrix = nuc4x4hb; gap opening penalty = 20, gap extension penalty = 5.

Various forms of the different embodiments of the invention, described herein, may be combined.

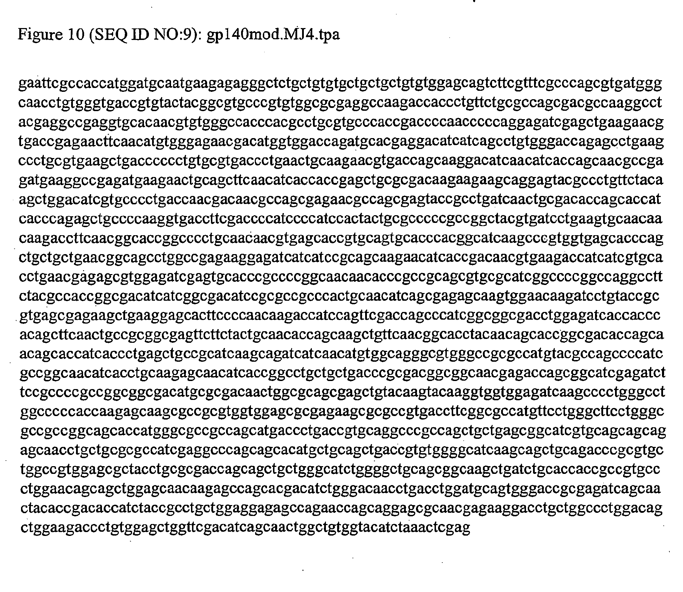

As noted above, Env-encoding constructs can be prepared using any of the full-length of gp160 constructs. For example, a gp140 form (Fig.10) was made by truncating gp160 (Fig.2) at nucleotide 2013; gp120 can be made by truncating gp160 (Fig.2) at nucleotide 1500 (Fig.2). Additional gp140 and gp120 forms can be made using the methods described herein. One or more stop codons may be typically added (e.g., nucleotides 2557 to 2559 of Fig.2). Further, the wild-type leader sequence can be modified and/or replaced with other leader sequences (e.g., TPA1 (TPA or tpa) leader sequences).

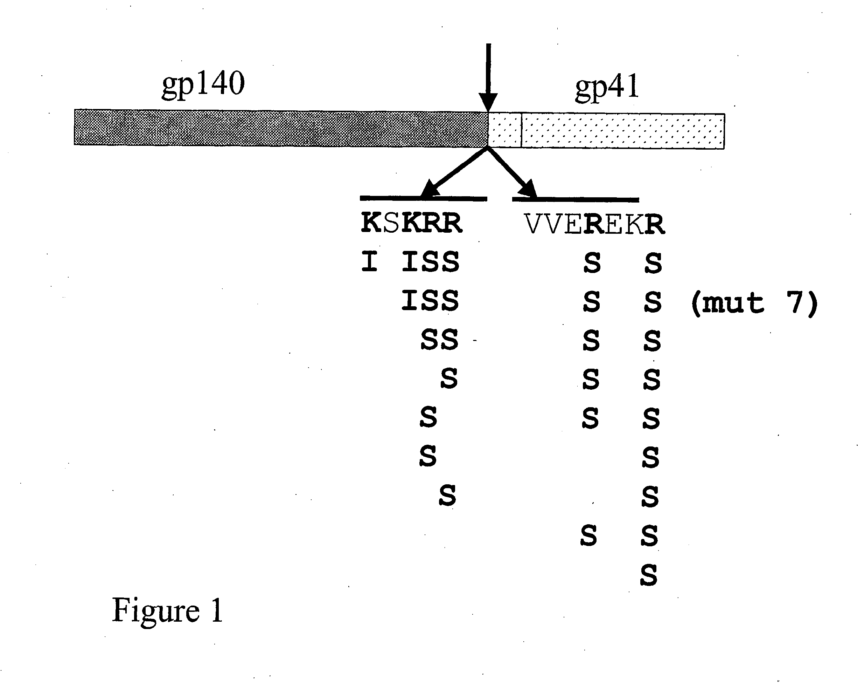

Thus, the polypeptide gp160 includes the coding sequences for gp120 and gp41. The polypeptide gp41 is comprised of several domains including, but not limited to an oligomerization domain (OD), a fusion peptide domain, and a transmembrane spanning domain (TM). (See, also, Table A, above). In the native envelope, the oligomerization domain is required for the non-covalent association of three gp41 polypeptides to form a trimeric structure: through non-covalent interactions with the gp41 trimer (and itself), the gp120 polypeptides are also organized in a trimeric structure. A cleavage site (or cleavage sites) exists approximately between the polypeptide sequences for gp 120 and the polypeptide sequences corresponding to gp41. This cleavage site(s) can be mutated to prevent cleavage at the site. The resulting gp140 polypeptide corresponds to a truncated form of gp160 where the transmembrane spanning domain of gp41 has been deleted. This gp140 polypeptide can exist in both monomeric and oligomeric (i.e. trimeric) forms by virtue of the presence of the oligomerization domain in the gp41 moiety. In the situation where the cleavage site has been mutated to prevent cleavage and the transmembrane portion of gp41 has been deleted the resulting polypeptide product is designated "mutated" gp140 (e.g., gp140.mut). As will be apparent to those in the field, the cleavage site can be mutated in a variety of ways, for example to abrogate protease and enhance expression of stable oligomeric forms. (Figure 1). In the constructs described herein (e.g., Fig.2-7 and 9 to 12) the mutation in the gp120/gp41 cleavage site changes the wild-type amino acid sequence KRRVVEREKR (SEQ ID NO:14) to ISSVVESEKS (SEQ ID NO:15). (See, also, Figure 1).

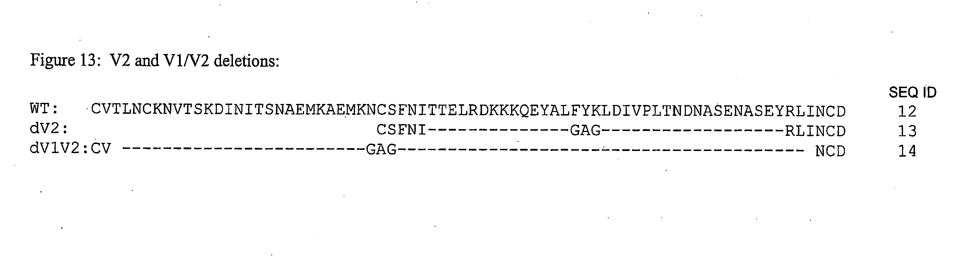

In yet other embodiments, hypervariable region(s) are deleted, N-glycosylation sites are modified, beta-sheet regions are modified and/or cleavage sites are mutated. Exemplary constructs having variable region deletions (V1 and/or V2), V2 deletes were constructed by deleting nucleotides from approximately 466 to approximately 571 (relative to Fig.2) and V1/V2 deletes were constructed by deleting nucleotides from approximately 372 to approximately 580 (relative to Fig.2). One or more amino acids may be inserted into the deleted regions, for example to help maintain the overall conformation of the Env protein. For instance, Figure 13 shows V2 and V1V2 deletes in which nucleotides encoding the three amino acid polypeptide GAG were added to the expression vectors such that GAG replaced the deleted region(s). The relative locations of V1 and/or V2 regions can also be readily determined by alignment to the regions shown in Table A.

It will be readily apparent that sequences derived from any HIV type C stain or clone can modified as described herein in order to achieve desirable modifications in that strain. Additionally, polyproteins can be constructed by fusing in-frame two or more polynucleotide sequences encoding polypeptide or peptide products. Further, polycistronic coding sequences may be produced by placing two or more polynucleotide sequences encoding polypeptide products adjacent each other, typically under the control of one promoter, wherein each polypeptide coding sequence may be modified to include sequences for internal ribosome binding sites.

The sequences of the present invention, for example, the modified (synthetic) polynucleotide sequences encoding HIV polypeptides, may be modified by deletions, point mutations, substitutions, frame-shifts, and/or further genetic modifications. Such modifications are taught generally in the art and may be applied in the context of the teachings of the present invention.

### EXAMPLE 2

### EXPRESSION ASSAYS FOR THE SYNTHETIC CODING SEQUENCES

The wild-type MJ4 Env-encoding sequences are cloned into expression vectors having the same features as the vectors into which the synthetic sequences are cloned.

Expression efficiencies for various vectors carrying the wild-type and synthetic sequences are evaluated as follows. Cells from several mammalian cell lines (293, RD, COS-7, and CHO; all obtained from the American Type Culture Collection, 10801 University Boulevard, Manassas, VA 20110-2209) are transfected with 2 µg of DNA in transfection reagent LT1 (PanVera Corporation, 545 Science Dr., Madison, WI). The cells are incubated for 5 hours in reduced serum medium (Opti-MEM, Gibco-BRL, Gaithersburg, MD). The medium is then replaced with normal medium as follows: 293 cells, IMDM, 10% fetal calf serum, 2% glutamine (BioWhittaker, Walkersville, MD); RD and COS-7 cells, D-MEM, 10% fetal calf serum, 2% glutamine (Opti-MEM, Gibco-BRL, Gaithersburg, MD); and CHO cells, Ham's F-12, 10% fetal calf serum, 2% glutamine (Opti-MEM, Gibco-BRL, Gaithersburg, MD). The cells are incubated for either 48 or 60 hours. Cell lysates are collected as described below in Example 3. Supernatants are harvested and filtered through 0.45 µm syringe filters. Supernatants are evaluated using the using 96-well plates coated with a rabbit polyclonal IgG directed against HIV Env. The HIV Env antigen binds to the coated wells. Biotinylated antibodies against HIV recognize the bound antigen. Conjugated strepavidin-horseradish peroxidase reacts with the biotin. Color develops from the reaction of peroxidase with TMB substrate. The reaction is terminated by addition of 4N H₂SO4. The intensity of the color is directly proportional to the amount of HIV antigen in a sample.

Synthetic MJ4 HIV Type C sequences (e.g., in expression cassettes) exhibit increased production of their protein products, relative to the wild-type MJ4 sequences, when expressed in a variety of cell lines.

### EXAMPLE 3

### WESTERN BLOT ANALYSIS OF EXPRESSION

Human 293 cells are transfected as described in Example 2 with pCMV-based vectors containing native or synthetic HIV Type C sequences. Cells are cultivated for 60 hours post-transfection. Supernatants are prepared as described. Cell lysates are prepared as follows. The cells are washed once with phosphate-buffered saline, lysed with detergent [1% NP40 (Sigma Chemical Co., St. Louis, MO) in 0.1 M Tris-HCl, pH 7.5], and the lysate transferred into fresh tubes. SDS-polyacrylamide gels (pre-cast 8-16%; Novex, San Diego, CA) are loaded with 20 µl of supernatant or 12.5 µl of cell lysate. A protein standard is also loaded (5 µl, broad size range standard; BioRad Laboratories, Hercules, CA). Electrophoresis is carried out and the proteins are transferred using a BioRad Transfer Chamber (BioRad Laboratories, Hercules, CA) to Immobilon P membranes (Millipore Corp., Bedford, MA) using the transfer buffer recommended by the manufacturer (Millipore), where the transfer is performed at 100 volts for 90 minutes. The membranes are exposed to HIV-1-positive human patient serum and immunostained using o-phenylenediamine dihydrochloride (OPD; Sigma).

Immunoblotting analysis shows that cells containing (expressing) the synthetic sequences produce the expected protein at higher per-cell concentrations than cells containing the native sequences. The proteins are seen in both cell lysates and supernatants. The levels of production are significantly higher in cell supernatants for cells transfected with the synthetic sequences of the present invention.

### EXAMPLE 4

### IN VIVO IMMUNOGENICITY OF SYNTHETIC HIV TYPE C SEQUENCES

### A. Immunization

To evaluate immunogenicity of the synthetic HIV Type C sequences described herein, mouse, rabbit and/or primate (e.g., macaques) studies are performed. The vector (e.g., plasmid) carrying the synthetic Env sequence, is diluted to the following final concentrations in a total injection volume of 100 µl: 20 µg, 2 µg, 0.2 µg, 0.02 and 0.002 µg. To overcome possible negative dilution effects of the diluted DNA, the total DNA concentration in each sample is brought up to 20 µg using the vector alone. As a control, DNA of the native Env is handled in the same manner. Twelve groups of four to ten Balb/c mice (Charles River, Boston, MA) are intramuscularly immunized (50 µl per leg, intramuscular injection into the tibialis anterior) according to the schedule in Table C.

**Table C**

| **Group** | **Gag or Env** | **Concentration of Env plasmid DNA (µg)** | **Immunized at time (weeks)¹:** |
|---|---|---|---|
| 1 | Synthetic | 20 | 0,4 |
| 2 | Synthetic | 2 | 0,4 |
| 3 | Synthetic | 0.2 | 0,4 |
| 4 | Synthetic | 0.02 | 0,4 |
| 5 | Synthetic | 0.002 | 0,4 |
| 6 | Synthetic | 20 | 0 |
| 7 | Synthetic | 2 | 0 |
| 8 | Synthetic | 0.2 | 0 |
| 9 | Synthetic | 0.02 | 0 |
| 10 | Synthetic | 0.002 | 0 |
| 11 | Native | 20 | 0,4 |
| 12 | Native | 2 | 0,4 |
| 13 | Native | 0.2 | 0,4 |
| 14 | Native | 0.02 | 0,4 |
| 15 | Native | 0.002 | 0,4 |
| 16 | Native | 20 | 0 |
| 17 | Native | 2 | 0 |
| 18 | Native | 0.2 | 0 |
| 19 | Native | 0.02 | 0 |
| 20 | Native | 0.002 | 0 |

| | | | |
|---|---|---|---|
| 1 = initial immunization at "week 0" | | | |

Groups 1-5 and 11-15 are bled at week 0 (before immunization), week 4, week 6, week 8, and week 12. Groups 6-20 and 16-20 are bled at week 0 (before immunization) and at week 4.

Similarly, groups of 5 rabbits or non human primates (macaques) are immunized with DNA encoding synthetic MJ4 sequences (optionally followed by boosting with the homologous protein structures). DNA for these studies are prepared using endotoxin-free Qiagen kits; small research batches of the engineered Env proteins are prepared by bulk transfection and small-scale purification. DNA immunizations are performed at 0, 4, and 12 weeks; the protein boost is given at 12 and 24 week time-points in rabbits. If necessary, an additional immunization with protein is administered at 36 weeks in rabbits. DNA immunization of non human primates are performed at 0, 4 and 24 weeks; the protein boost is given at 24 and 36 week time points in non human primates. Animals are bled prior to the start of the immunizations and at 2-week intervals.

One or more DNA immunizations are performed by needle injection of naked DNA in saline or DNA absorbed to PLG, with or without electroporation (essentially as described in Dupuis et al. (2000) J Immunol 165(5):2850-8; Widera et al. (2000) J Immunol 164(9):4635-40). Alternatively, DNA is delivered using alphavirus replicon particles. Antibody titers are substantially increased and the dose of DNA required to prime responses is markedly reduced by electroporation. Electroporation appears to be a highly efficient method for the priming of both neutralizing antibody and Env-specific CD8+ CTL in macaques. Thus, electroporation is used to efficiently deliver Env DNAs (e.g., plasmids) to rabbits and non-human primates. The DNA prime/protein boost strategy of immunization that will be used here allows for screening of multiple Env structures in rabbits and non-human primates (*e.g.,* macaques or baboons) with the potential for epitope presentation *in situ* in the host when delivered as DNA vaccines.

### B. Humoral Immune Response

The humoral immune response is checked with ELISAs (enzyme-linked immunosorbent assays) of the mice sera at biweekly or 4 week intervals after each immunization. Similarly, rabbits and non-human primates are evaluated at the corresponding post-immunization timepoints. The immune responses are compared to pre-bleed sera (negative controls).

The antibody titers of the sera are determined by using anti-Env antibody ELISAs. Briefly, sera from immunized animals are screened for antibodies directed against the HIV Env protein(s). ELISA microtiter plates are coated with 0.2 µg of HIV protein per well overnight and washed four times; subsequently, blocking is done with PBS-0.2% Tween (Sigma) for 2 hours. After removal of the blocking solution, 100 µl of diluted immune test serum is added. Sera are tested at 1/25 dilutions and by serial 3-fold dilutions, thereafter. Microtiter plates are washed four times and incubated with a secondary, peroxidase-coupled anti-mouse IgG antibody (Pierce, Rockford, IL). ELISA plates are washed and 100 µl of 3, 3', 5, 5'-tetramethyl benzidine (TMB; Pierce) is added per well. The optical density of each well is measured after 15 minutes. The titers reported are the reciprocal of the dilution of serum that gave a half-maximum optical density (O.D.).

Neutralization assays are performed using PHA-activated human PBMC as target cells according to standard procedures. All HIV-1 isolates are grown in human PBMCs, aliquoted and kept frozen at -80°C until further use. Viruses (50-300 TCID₅₀ in 50 µl are pre-incubated with an equal volume of serially diluted heat-inactivated (35 minutes at 56°C) macaque sera for one hour at 37°C, in 96 (U-bottom) well plates. For each serum dilution, triplicate wells are used. Pre-immunization sera from the same animals are also incubated with the viruses and serve as controls. To each well, an equal volume (100 µl) of cells (0.4 x 10⁶ per well) is added. Following an overnight incubation, the sera and the remaining inoculum is removed by cell washing. The Env antigen concentration in each well is evaluated during exponential viral growth, usually seven to ten days later, using commercially available HIV Env-antigen kits (Coulter). The percent of virus inhibition for each serum dilution is determined at the peak of viral replication as: (control - experimental / control) x 100, where control stands for the HIV Env antigen concentration in the presence of pre-immunization serum and experimental is the concentration in the presence of post-immunization sera.

Synthetic sequences (*e.g*., expression cassettes) will provide improved immunogenicity relative to the native sequences and, in addition, prime for the production of neutralizing antibodies.

### C. Cellular Immune Response

The following assays for measurement of cellular immune responses are used in the analysis of HIV compositions described in herein in animals (*e.g.,* rhesus macaques): 1) CTL bulk culture and ⁵¹Cr-release, 2) lymphoproliferation, 3) intracellular cytokine flow cytometry, and 4) ELISPOT. (See, also, zur Megede et al. (2000) J. Vinol. 74:2628-2635 (describing ICC); Cherpelis et al. (2001) Immunol. Lett. 79:47-55 (describing LPA assays); and Vajdy et al. (2001) J. Infect Dis 15;184(12):1613-1616 (describing ELISPOT)). Reagents such as recombinant Gag and Env proteins and peptides are available as well as the required monoclonal antibodies to perform these assays.

For example, the frequency of specific cytotoxic T-lymphocytes (CTL) is evaluated by a standard chromium release assay of peptide pulsed mouse (Balb/c, CB6F1 and/or C3H) CD4 cells. HIV polypeptide (*e.g*., Env) expressing vaccinia virus infected CD-8 cells are used as a positive control. Briefly, spleen cells (Effector cells, E) are obtained from the mice immunized as described above are cultured, restimulated, and assayed for CTL activity against Gag peptide-pulsed target cells as described (Doe, B., and Walker, C.M., AIDS 10(7):793-794, 1996). Cytotoxic activity is measured in a standard 51Cr release assay. Target (T) cells are cultured with effector (E) cells at various E:T ratios for 4 hours and the average cpm from duplicate wells are used to calculate percent specific 51Cr release.

Cytotoxic T-cell (CTL) activity is measured in splenocytes recovered from the mice immunized with HIV Env DNA. Effector cells from the Env DNA-immunized animals exhibit specific lysis of HIV polypeptide-pulsed SV-BALB (MHC matched) targets cells, indicative of a CTL response. Target cells that are peptide-pulsed and derived from an MHC-unmatched mouse strain (MC57) are not lysed.

Thus, synthetic sequences exhibit increased potency for induction of cytotoxic T-lymphocyte (CTL) responses by DNA immunization.

### EXAMPLE 5

### CROSS-STRAIN NEUTRALIZING ANTIBODIES

### A. Neutralization of HIV subtype B following prime boost

Five rabbits each were immunized with three DNA samples at 0, 4 and 12 weeks. Three different groups of rabbits were immunized with 1 mg of plasmid DNA synthetic MJ4 gp140 (Rabbits 1-5), gp140 (deletion V2) (Rabbits 6-10) or gp 140 (deletion V1V2) (Rabbits 11-15). Boost was with 50 micrograms of the corresponding oligomeric envelope protein (i.e., oligomeric gp140, oligomeric gp 140 deletion V2 or oligomeric gp 140 deletion V1V2). Immunization protocols were as follows:

### Immunization 1-3: Plasmid DNA /Saline immunization

The immunogen was provided at 1.0mg/ml total DNA in sterile 0.9% saline and stored at -80°C until use. DNA was thawed at room temperature such that the material was clear or slightly opaque, with no particulate matter. Each rabbit was immunized by needle injection with 0.25ml DNA mixture per side (2 sites IM/Quadriceps and 2 sites IM/Gluteus), 1.0ml per animal.

### Immunization 3-4: Protein Immunization + MF59

Protein doses are 50ug protein per animal. The initial protein was diluted to 0.100 mg/ml in buffer containing 10mM NaCitrate and 300mM NaCl (pH 6.0) and stored at - 80°C until use. Prior to administration, protein was thawed at room temperature so that material was clear with no particulate matter. An equal volume of MF59C adjuvant was added to the thawed protein and mixed by inverting the tube. Protein-MF59C materials were used within one hour of mixing. Each rabbit was immunized by needle injection with 0.5ml adjuvanted protein per side, IM/Glut for a total of 1ml per animal.

Sera were collected at week 14 and were tested for virus neutralizing activity against HIV-1 SF-162. Neutralizing antibody is shown as reciprocal of endpopint dilution showing 50% virus inhibition versus control. Neutralization in M7-Luc cells was also tested. Values are the serum dilution at which relative luminescence units (RLU) were reduced 50% compared to virus control wells (no test sample). Values are the serum dilution at which relative luminescence units (RLU) were reduced 50% compared to virus control wells (no test sample). Values greater than 100 are considered positive for virus neutralization. Results are shown in the Table 1.

| **TABLE 1** | | |
|---|---|---|
| **Group** | **Rabbit Number** | **Neutralizing Ab** |
| gp140 | 1 | 667 |
| | 2 | 244 |
| | 3 | (67) |
| | 4 | 955 |
| | 5 | 303 |
| gp140delV2 | 6 | 526 |
| | 7 | 239 |
| | 8 | 1206 |
| | 9 | (43) |
| | 10 | Not available |
| Gp140delV1V2 | 11 | 327 |
| | 12 | 2818 |
| | 13 | 671 |
| | 14 | 4576 |
| | 15 | 1684 |

Thus, the synthetic sequences described herein induce the production of antibodies that neutralize HIV subtype B.

## Claims

1. A polynucleotide sequence encoding a polypeptide including an HIV Env polypeptide, wherein said HIV Env polypeptide is gp120, gp140 or gp160 and comprises a deletion of the V2 region or a deletion of the V 1 and V2 regions, and wherein the polynucleotide sequence encoding said Env polypeptide has at least 90% sequence identity to the sequence presented in any one of Figures 3-4, 6-8 and 11-12, and wherein the encoded polypeptide is capable of inducing the production of antibodies that neutralise HIV subtype B.

2. The polynucleotide sequence of claim 1, wherein the sequence has at least 95% identity to the sequence presented in any of Figures 3-4, 6-8 and 11-12.

3. The polynucleotide sequence of claim 1, wherein the sequence is selected from the group consisting of the sequences presented in Figure 3, Figure 4, Figure 6, Figure 7, Figure 8, Figure 11 and Figure 12.

4. An expression cassette comprising the polynucleotide sequence of claim 1.

5. The expression cassette of claim 4, wherein said polynucleotide sequence further includes a polynucleotide sequence encoding an additional polypeptide.

6. The expression cassette of claim 5, wherein the additional polypeptide is selected from the group consisting of viral proteins, such as an HIV protein selected from the group consisting of Gag, Pol, vif, vpr, tat, rev, vpu and nef, and cytokines.

7. The expression cassette of claim 6, wherein the HIV proteins are from at least two different HIV subtypes.

8. A recombinant expression system for use in a selected host cell, comprising, an expression cassette of claim 4, and wherein said polynucleotide sequence is operably linked to control elements compatible with expression in the selected host cell.

9. The recombinant expression system of claim 8, wherein said control elements are selected from the group consisting of a transcription promoter, a transcription enhancer element, a transcription termination signal, polyadenylation sequences, sequences for optimization-of initiation of translation, and translation termination sequences.

10. The recombinant expression system of claim 9, wherein said transcription promoter is selected form the group consisting of CMV, CMV+intron A, SV40, RSV, HIV-Ltr, MMLV-ltr, and metallothionein.

11. A cell comprising an expression cassette of claim 4, and wherein said polynucleotide sequence is operably linked to control elements compatible with expression in the selected cell.

12. The cell of claim 11, wherein the cell is a mammalian cell selected from the group consisting of BHK, VERO, HT1080, 293, RD, COS-7, and CHO cells.

13. The cell of claim 11, wherein the cell is an insect cell such as Trichoplusia ni (Tn5) or Sf9.

14. The cell of claim 11, wherein the cell is a bacterial cell, a yeast cell or a plant cell.

15. The cell of claim 11, wherein the cell is an antigen presenting cell such as a lymphoid cell selected from the group consisting of macrophage, monocytes, dendritic cells, B-cells, T-cells, stem cells, and progenitor cells thereof.

16. The cell of claim 11, wherein the cell is a primary cell, an immortalized cell or a tumor-derived cell.

17. A cell line useful for packaging lentivirus vectors, comprising suitable host cells that have been transfected with an expression vector containing an expression cassette of claim 4, and wherein said polynucleotide sequence is operably linked to control elements compatible with expression in the host cell.

18. A gene delivery vector for use in a mammalian subject, comprising a suitable gene delivery vector for use in said subject, wherein the vector comprises an expression cassette of claim 4, and wherein said polynucleotide sequence is operably linked to control elements compatible with expression in the subject.

19. A gene delivery vector comprising an alphavirus vector construct wherein said alphavirus construct comprises an expression cassette according to claim 4.

20. The gene delivery vector of claim 19, wherein the alphavirus vector construct is a cDNA vector construct.

21. The gene delivery vector of claim 20, wherein the alphavirus comprises a recombinant alphavirus particle preparation.

22. The gene delivery vector of claim 19, wherein the vector comprises a eukaryotic layered vector initiation system.

23. A gene delivery vector of claim 18 introduced into said subject under conditions that are compatible with expression of said expression cassette in said subject, for use in a method of DNA immunization.

24. The gene delivery vector of claim 23, wherein said gene delivery vector is a nonviral vector.

25. The gene delivery vector of claim 24, wherein said vector is delivered using a particulate carrier or said vector is coated on a gold or tungsten particle and said coated particle is delivered to said subject using a gene gun or said vector is encapsulated in a liposome preparation.

26. The gene delivery vector of claim 23, wherein said vector is a viral vector such as a retroviral vector, or a lentiviral vector.

27. The gene delivery vector of claim 23, wherein said subject is a mammal such as a human.

28. A polypeptide encoded by a polynucleotide sequence according to any one of claims 1-3.

29. A composition comprising:
(i) a polynucleotide sequence according to any of claims 1-3;
(ii) an expression cassette according to any of claims 4-7;
(iii) a gene delivery vector according to any of claims 18-27; or
(iv) a polypeptide according to claim 28,
and one more pharmaceutically acceptable excipients or vehicles.

30. A polynucleotide sequence according to any of claims 1-3, an expression cassette according to any of claims 4-7, a gene delivery vector according to any of claims 18-27, or a polypeptide according to claim 28, for use in generating an immune response in a subject.

31. Use of a polynucleotide sequence according to any of claims 1-3, an expression cassette according to any of claims 4-7, a gene delivery vector according to any of claims 18-27, or a polypeptide according to claim 28, in the manufacture of a medicament for generating an immune response in a subject.

32. The gene delivery vector of claim 30 or the use of claim 31, wherein said gene delivery vector is a nonviral vector.

33. The gene delivery vector of claim 30 or the use of claim 31, wherein said gene delivery vector is delivered using as particulate carrier, or said vector is coated on a gold or tungsten particle and said coated particle is delivered to said vertebrate cell using a gene gun or said vector is encapsulated in a liposome preparation.

34. The gene delivery vector of claim 30 or the use of claim 31, wherein said gene delivery vector is a viral vector such as a retroviral vector, or a lentiviral vector.

35. The polynucleotide sequence, expression cassette, gene delivery vector or polypeptide according to claim 30, or the use according to claim 31, wherein said subject is a mammal such as a human.

36. The polynucleotide sequence, expression cassette, gene delivery vector or polypeptide according to claim 30, or the use according to claim 31, where said immune response is a humoral immune response, or a cellular immune response.

37. The gene delivery vector according to claim 30, or the use according to claim 31, wherein the gene delivery vector is administered intramuscularly, intramucosally, intranasally, subcutaneously, intradermally, transdermally, intravaginally, intrarectally, orally or intravenously.

## Patentansprüche

1. Polynukleotidsequenz, die für ein Polypeptid einschließlich eines HIV-Env-Polypeptids codiert, wobei das HIV-Env-Polypeptid gp120, gp140 oder gp160 ist und eine Deletion der V2-Region oder eine Deletion der V1- und V2-Regionen umfasst, und wobei die Polynukleotidsequenz, die für das Env-Polypeptid codiert, mindestens 90% Sequenzidentität zu der Sequenz gemäß einer der Figuren 3-4, 6-8 und 11-12 aufweist, und wobei das codierte Polypeptid fähig ist, die Produktion von Antikörpern, die HIV Subtyp B neutralisieren, zu induzieren.

2. Polynukleotidsequenz nach Anspruch 1, wobei die Sequenz mindestens 95% Identität zu der Sequenz gemäß einer der Figuren 3-4, 6-8 und 11-12 aufweist.

3. Polynukleotidsequenz nach Anspruch 1, wobei die Sequenz aus der Gruppe bestehend aus den Sequenzen gemäß Fig. 3, Fig. 4, Fig. 6, Fig. 7, Fig. 8, Fig. 11 und Fig. 12 ausgewählt ist.

4. Expressionskassette, die die Polynukleotidsequenz nach Anspruch 1 umfasst.

5. Expressionskassette nach Anspruch 4, wobei die Polynukleotidsequenz weiterhin eine Polynukleotidsequenz, die für ein zusätzliches Polypeptid codiert, beinhaltet.

6. Expressionskassette nach Anspruch 5, wobei das zusätzliche Polypeptid aus der Gruppe bestehend aus Virusproteinen, wie ein HIV-Protein, ausgewählt aus der Gruppe bestehend aus Gag, Pol, vif, vpr, tat, rev, vpu und nef, und Cytokinen ausgewählt ist.

7. Expressionskassette nach Anspruch 6, wobei die HIV-Proteine von mindestens zwei unterschiedlichen HIV-Subtypen stammen.

8. Rekombinantes Expressionssystem zur Verwendung in einer ausgewählten Wirtszelle, die Folgendes umfasst: eine Expressionskassette nach Anspruch 4, und wobei die Polynukleotidsequenz operativ mit Kontrollelementen, die mit der Expression in der ausgewählten Wirtszelle kompatibel sind, verknüpft ist.

9. Rekombinantes Expressionssystem nach Anspruch 8, wobei die Kontrollelemente aus der Gruppe bestehend aus einem Transkriptionspromoter, einem Transkriptions-Enhancer-Element, einem Transkriptionsterminationssignal, Polyadenylierungssequenzen, Sequenzen für die Optimierung der Translationsinitiation sowie Translationsterminationssequenzen ausgewählt sind.

10. Rekombinantes Expressionssystem nach Anspruch 9, wobei der Transkriptionspromoter aus der Gruppe bestehend aus CMV, CMV+ Intron A, SV40, RSV, HIV-ltr, MMLV-ltr und Metallothionein ausgewählt ist.

11. Zelle, die eine Expressionskassette nach Anspruch 4 umfasst, wobei die Polynukleotidsequenz operativ mit Kontrollelementen, die mit der Expression in der ausgewählten Zelle kompatibel sind, verknüpft ist.

12. Zelle nach Anspruch 11, wobei es sich bei der Zelle um eine Säugetierzelle, ausgewählt aus der Gruppe bestehend aus BHK-, VERO-, HT1080-, 293-, RD-, COS-7- und CHO-Zellen, handelt.

13. Zelle nach Anspruch 11, wobei es sich bei der Zelle um eine Insektenzelle wie Trichoplusia ni (Tn5) oder Sf9 handelt.

14. Zelle nach Anspruch 11, wobei es sich bei der Zelle um eine Bakterienzelle, eine Hefezelle oder eine Pflanzenzelle handelt.

15. Zelle nach Anspruch 11, wobei es sich bei der Zelle um eine antigenpräsentierende Zelle, wie eine Lymphoidzelle, ausgewählt aus der Gruppe bestehend aus Makrophage, Monozyten, Dendritenzellen, B-Zellen, T-Zellen, Stammzellen und ihren Vorläuferzellen, handelt.

16. Zelle nach Anspruch 11, wobei es sich bei der Zelle um eine primäre Zelle, eine immortalisierte Zelle oder eine von einem Tumor abstammende Zelle handelt.

17. Zelllinie mit Eignung für das Verpacken von Lentivirus-Vektoren, umfassend geeignete Wirtszellen, die mit einem Expressionsvektor, der eine Expressionskassette nach Anspruch 4 enthält, transfiziert worden sind, wobei die Polynukleotidsequenz operativ mit Kontrollelementen, die mit der Expression in der Wirtszelle kompatibel sind, verknüpft ist.

18. Genabgabevektor zur Verwendung in einem Säugetiersubjekt, der einen geeigneten Genabgabevektor zur Verwendung in dem Subjekt umfasst, wobei der Vektor eine Expressionskassette nach Anspruch 4 umfasst und wobei die Polynukleotidsequenz operativ mit Kontrollelementen, die mit der Expression in dem Subjekt kompatibel sind, verknüpft ist.

19. Genabgabevektor, der ein Alphavirus-Vektorkonstrukt umfasst, wobei das Alphavirus-Konstrukt eine Expressionskassette nach Anspruch 4 umfasst.

20. Genabgabevektor nach Anspruch 19, wobei es sich bei dem Alphavirus-Vektorkonstrukt um ein cDNA-Vektorkonstrukt handelt.

21. Genabgabevektor nach Anspruch 20, wobei das Alphavirus ein rekombinantes Alphaviruspartikelpräparat umfasst.

22. Genabgabevektor nach Anspruch 19, wobei der Vektor ein eukaryontisches geschichtetes Vektorinitiationssystem umfasst.

23. Genabgabevektor nach Anspruch 18, der in das Subjekt unter Bedingungen eingeführt wird, die mit der Expression der Expressionskassette in dem Subjekt kompatibel sind, zur Verwendung in einem Verfahren zur DNA-Immunisierung.

24. Genabgabevektor nach Anspruch 23, wobei es sich bei dem Genabgabevektor um einen Nichtvirusvektor handelt.

25. Genabgabevektor nach Anspruch 24, wobei der Vektor unter Verwendung eines teilchenförmigen Trägers abgegeben wird oder der Vektor auf ein Gold- oder Wolframpartikel aufgetragen wird und das beschichtete Partikel in das Subjekt unter Verwendung einer Genkanone abgegeben wird oder der Vektor in einem Liposompräparat verkapselt ist.

26. Genabgabevektor nach Anspruch 23, wobei es sich bei dem Vektor um einen Virusvektor wie einen Retrovirusvektor oder einen Lentivirusvektor handelt.

27. Genabgabevektor nach Anspruch 23, wobei es sich bei dem Subjekt um ein Säugetier, wie einen Menschen, handelt.

28. Polypeptid, das von einer Polynukleotidsequenz nach einem der Ansprüche 1-3 codiert wird.

29. Zusammensetzung, die Folgendes umfasst:
(i) eine Polynukleotidsequenz nach einem der Ansprüche 1-3;
(ii) eine Expressionskassette nach einem der Ansprüche 4-7;
(iii)einen Genabgabevektor nach einem der Ansprüche 18-27; oder
(iv) ein Polypeptid nach Anspruch 28,
sowie ein oder mehr pharmazeutisch unbedenkliche Konstituentien.

30. Polynukleotidsequenz nach einem der Ansprüche 1-3, Expressionskassette nach einem der Ansprüche 4-7, Genabgabevektor nach einem der Ansprüche 18-27 oder Polypeptid nach Anspruch 28 für die Verwendung bei der Erzeugung einer Immunantwort in einem Subjekt.

31. Verwendung einer Polynukleotidsequenz nach einem der Ansprüche 1-3, Expressionskassette nach einem der Ansprüche 4-7, Genabgabevektor nach einem der Ansprüche 18-27 oder Polypeptid nach Anspruch 28, in der Herstellung eines Arzneimittels zur Erzeugung einer Immunantwort in einem Subjekt.

32. Genabgabevektor nach Anspruch 30 oder Verwendung nach Anspruch 31, wobei es sich bei dem Genabgabevektor um einen Nichtvirusvektor handelt.

33. Genabgabevektor nach Anspruch 30 oder Verwendung nach Anspruch 31, wobei es sich bei dem Genabgabevektor unter Verwendung eines teilchenförmigen Trägers abgegeben wird oder der Vektor auf ein Gold- oder Wolframpartikel aufgetragen wird und das beschichtete Partikel in die Wirbeltierzelle unter Verwendung einer Genkanone abgegeben wird oder der Vektor in einem Liposompräparat verkapselt ist.

34. Genabgabevektor nach Anspruch 30 oder Verwendung nach Anspruch 31, wobei es sich bei dem Genabgabevektor um einen Virusvektor wie einen Retrovirusvektor oder einen Lentivirusvektor handelt.

35. Polynukleotidsequenz, Expressionskassette, Genabgabevektor oder Polypeptid nach Anspruch 30 oder Verwendung nach Anspruch 31, wobei es sich bei dem Subjekt um ein Säugetier, wie einen Menschen, handelt.

36. Polynukleotidsequenz, Expressionskassette, Genabgabevektor oder Polypeptid nach Anspruch 30 oder Verwendung nach Anspruch 31, wobei es sich bei der Immunantwort um eine humorale Immunantwort oder eine zelluläre Immunreaktion handelt.

37. Genabgabevektor nach Anspruch 30 oder Verwendung nach Anspruch 31, wobei der Genabgabevektor intramuskulär, intramucosal, intranasal, subkutan, intradermal, transdermal, intravaginal, intrarektal, oral oder intravenös verabreicht wird.

## Revendications

1. Séquence polynucléotidique codant pour un polypeptide comprenant un polypeptide Env du VIH, ledit polypeptide Env du VIH étant le gp120, le gp140 ou le gp160 et comprenant une délétion de la région V2 ou une délétion des régions V1 et V2, et la séquence polynucléotidique codant pour ledit polypeptide Env ayant une identité de séquence d'au moins 90 % avec la séquence présentée dans l'une quelconque des Figures 3-4, 6-8 et 11-12, le polypeptide codé étant capable d'induire la production d'anticorps qui neutralisent le sous-type B du VIH.

2. Séquence polynucléotidique de la revendication 1, la séquence ayant une identité d'au moins 95 % avec la séquence présentée dans l'une quelconque des Figures 3-4, 6-8 et 11-12.

3. Séquence polynucléotidique de la revendication 1, la séquence étant choisie dans le groupe consistant en les séquences présentées sur la Figure 3, la Figure 4, la Figure 6, la Figure 7, la Figure 8, la Figure 11 et la Figure 12.

4. Cassette d'expression comprenant la séquence polynucléotidique de la revendication 1.

5. Cassette d'expression de la revendication 4, dans laquelle ladite séquence polynucléotidique comprend en outre une séquence polynucléotidique codant pour un polypeptide additionnel.

6. Cassette d'expression de la revendication 5, dans laquelle le polypeptide additionnel est choisi dans le groupe consistant en les protéines virales, telles qu'une protéine du VIH choisie dans le groupe consistant en Gag, Pol, vif, vpr, tat, rev, vpu et nef, et les cytokines.

7. Cassette d'expression de la revendication 6, dans laquelle les protéines du VIH proviennent d'au moins deux sous-types différents du VIH.

8. Système d'expression recombinant pour utilisation dans une cellule hôte sélectionnée, comprenant une cassette d'expression de la revendication 4, et dans lequel ladite séquence polynucléotidique est liée de manière opérationnelle à des éléments régulateurs compatibles avec une expression dans la cellule hôte sélectionnée.

9. Système d'expression recombinant de la revendication 8, dans lequel lesdits éléments régulateurs sont choisis dans le groupe consistant en un promoteur de transcription, un élément amplificateur de transcription, un signal de terminaison de transcription, des séquences de polyadénylation, des séquences pour l'optimisation de l'initiation de la traduction, et des séquences de terminaison de traduction.

10. Système d'expression recombinant de la revendication 9, dans lequel ledit promoteur de transcription est choisi dans le groupe consistant en le CMV, le CMV+intron A, le SV40, le RSV, le HIV-Ltr, le MMLV-Ltr et la métallothionéine.

11. Cellule comprenant une cassette d'expression de la revendication 4, et dans laquelle ladite séquence polynucléotidique est liée de manière opérationnelle à des éléments régulateurs compatibles avec une expression dans la cellule sélectionnée.

12. Cellule de la revendication 11, la cellule étant une cellule de mammifère, choisie dans le groupe consistant en les cellules BHK, VERO, HT1080, 293, RD, COS-7 et CHO.

13. Cellule de la revendication 11, la cellule étant une cellule d'insecte telle que Trichoplusia ni (Tn5) ou Sf9.

14. Cellule de la revendication 11, la cellule étant une cellule bactérienne, une cellule de levure ou une cellule végétale.

15. Cellule de la revendication 11, la cellule étant une cellule présentant l'antigène, telle qu'une cellule lymphoïde choisie dans le groupe consistant en les macrophages, les monocytes, les cellules dendritiques, les cellules B, les cellules T, les cellules souches et les cellules progénitrices de celles-ci.

16. Cellule de la revendication 11, la cellule étant une cellule primaire, une cellule immortalisée ou une cellule dérivant d'une tumeur.

17. Lignée cellulaire pour encapsidation de vecteurs lentiviraux, comprenant des cellules hôtes appropriées qui ont été transfectées avec un vecteur d'expression contenant une cassette d'expression de la revendication 4, ladite séquence polynucléotidique étant liée de manière opérationnelle à des éléments régulateurs compatibles avec une expression dans la cellule hôte.

18. Vecteur de délivrance de gènes pour utilisation chez un sujet mammifère, comprenant un vecteur approprié de délivrance de gènes pour utilisation chez ledit sujet, ledit vecteur comprenant une cassette d'expression de la revendication 4, et ladite séquence polynucléotidique étant liée de manière opérationnelle à des éléments régulateurs compatibles avec une expression chez le sujet.

19. Vecteur de délivrance de gènes comprenant une construction de vecteur alpha-viral, ladite construction alpha-virale comprenant une cassette d'expression selon la revendication 4.

20. Vecteur de délivrance de gènes de la revendication 19, dans lequel ladite construction de vecteur alpha-viral est une construction d'ADNc vecteur.

21. Vecteur de délivrance de gènes de la revendication 20, dans lequel l'alpha-virus comprend une préparation de particules alpha-virales recombinantes.

22. Vecteur de délivrance de gènes de la revendication 19, le vecteur comprenant un système d'initiation de vecteur multicouche eucaryote ("eukaryotic layered vector initiation system").

23. Vecteur de délivrance de gènes de la revendication 18, introduit dans ledit sujet dans des conditions qui sont compatibles avec une expression de ladite cassette d'expression chez ledit sujet, pour utilisation dans un procédé d'immunisation d'ADN.

24. Vecteur de délivrance de gènes de la revendication 23, ledit vecteur de délivrance de gènes étant un vecteur non viral.

25. Vecteur de délivrance de gènes de la revendication 24, ledit vecteur étant délivré par utilisation d'un support particulaire, ou ledit vecteur étant appliqué sur une particule d'or ou de tungstène, et ladite particule revêtue étant délivrée audit sujet par utilisation d'un canon à gènes, où ledit vecteur est encapsulé dans une préparation de liposomes.

26. Vecteur de délivrance de gènes de la revendication 23, ledit vecteur étant un vecteur viral tel qu'un vecteur rétroviral ou un vecteur lentiviral.

27. Vecteur de délivrance de gènes de la revendication 23, pour lequel ledit sujet est un mammifère, tel qu'un humain.

28. Polypeptide codé par une séquence polynucléotidique selon l'une quelconque des revendications 1 à 3.

29. Composition comprenant :
(i) une séquence polynucléotidique selon l'une quelconque des revendications 1-3 ;
(ii) une cassette d'expression selon l'une quelconque des revendications 4-7 ;
(iii) un vecteur de délivrance de gènes selon l'une quelconque des revendications 18-27 ; ou
(iv) un polypeptide selon la revendication 28,
et un ou plusieurs excipients ou véhicules pharmaceutiquement acceptables.

30. Séquence polynucléotidique selon l'une quelconque des revendications 1-3, cassette d'expression selon l'une quelconque des revendications 4-7, vecteur de délivrance de gènes selon l'une quelconque des revendications 18-27, ou polypeptide selon la revendication 28, pour utilisation dans la production d'une réponse immune chez un sujet.

31. Utilisation d'une séquence polynucléotidique selon l'une quelconque des revendications 1-3, d'une cassette d'expression selon l'une quelconque des revendications 4-7, d'un vecteur de délivrance de gènes selon l'une quelconque des revendications 18-27, ou d'un polypeptide selon la revendication 28, pour la fabrication d'un médicament destiné à produire une réponse immune chez un sujet.

32. Vecteur de délivrance de gènes de la revendication 30 ou utilisation de la revendication 31, ledit vecteur de délivrance de gènes étant un vecteur non viral.

33. Vecteur de délivrance de gènes de la revendication 30 ou utilisation de la revendication 31, ledit vecteur de délivrance de gènes étant délivré par utilisation d'un support particulaire, ou ledit vecteur étant appliqué sur une particule d'or ou de tungstène et ladite particule revêtue étant délivrée à ladite cellule de vertébré par utilisation d'un canon à gènes, ou ledit vecteur étant encapsulé dans une préparation de liposomes.

34. Vecteur de délivrance de gènes de la revendication 30 ou utilisation de la revendication 31, ledit vecteur de délivrance de gènes étant un vecteur viral, tel qu'un vecteur rétroviral ou un vecteur lentiviral.

35. Séquence polynucléotidique, cassette d'expression, vecteur de délivrance de gènes ou polypeptide selon la revendication 30, ou utilisation selon la revendication 31, ledit sujet étant un mammifère, tel qu'un humain.

36. Séquence polynucléotidique, cassette d'expression, vecteur de délivrance de gènes ou polypeptide selon la revendication 30, ou utilisation selon la revendication 31, ladite réponse immune étant une réponse immune humorale ou une réponse immune cellulaire.

37. Vecteur de délivrance de gènes selon la revendication 30, ou utilisation selon la revendication 31, le vecteur de délivrance des gènes étant administré par voie intramusculaire, intramuqueuse, intranasale, sous-cutanée, intradermique, transdermique, intravaginale, intrarectale, orale ou intraveineuse.
